# EUROPEAN PATENT APPLICATION

(11) **EP 4 707 396 A1**
(43) Date of publication of application: **11.03.2026**
(21) Application number: 24198648.8
(22) Date of filing: 05.09.2024
(51) Int. Cl.: C12N 15/63, C07K 14/47, C12N 15/85

(54) **METHOD AND EXPRESSION SYSTEM FOR PRODUCING BIOLOGICALLY ACTIVE MOLECULES**

(71) Applicant: Ningaloo Biosystems GmbH, 51105 Köln (DE); Rheinische Friedrich-Wilhelms-Universität Bonn, 53113 Bonn (DE)
(72) Inventor: WACHTEN, Dagmar, 50354 Hürth (DE); GEBEL, Jeannette, 40764 Langenfeld (DE); STAHL, Rainer, 53343 Wachtberg (DE); DUTHIE, Fraser, 50733 Köln (DE); SCHMIDT, Hanns-Martin, 50733 Köln (DE); CIGLIERI, Elisa, 50674 Köln (DE); MÜLLER-HARTMANN, Herbert, 50937 Köln (DE)
(74) Representative: Remus, Alvaro Johannes

(57) **Abstract**

The invention relates to a method, expression system and related kit for producing at least one biologically active molecule, in particular a biotherapeutic, by optogenetic control of gene expression in at least one eukaryotic or prokaryotic cell, wherein the biologically active molecule comprises at least one protein of interest comprising at least two different subunits. In particular, one embodiment of the expression system according to the invention comprises three expression cassettes 2, 3, 4 for optogenetic control of the production of a protein of interest comprising two subunits 5, 6. A first expression cassette 2 comprises a first nucleic acid sequence 7 that is operably linked to a first promoter 8 and encodes a photosensitive transcription factor 9 comprising a gene expression controlling domain 10. The expression system 1 further comprises a second expression cassette 3 comprising a second nucleic acid sequence 11 encoding the first subunit 5 of the protein of interest. The second nucleic acid sequence 11 is operably linked to a second promoter 12 being operably linked to a transcription factor binding site 13 designed to bind the photosensitive transcription factor 9. The expression system 1 also comprises a third expression cassette 4 comprising a third nucleic acid sequence 15 encoding the second subunit 6 of the protein of interest and being operably linked to a third promoter 16.

## Description

### Field of the invention:

The invention relates to a method, expression system and related kit for producing at least one biologically active molecule, in particular a biotherapeutic, by optogenetic control of gene expression in at least one eukaryotic or prokaryotic cell, wherein the biologically active molecule comprises at least one protein of interest comprising at least two different subunits. In particular, the invention concerns biological production systems, primarily based on living cells cultivated in bioreactors or other culture vessels, and more particularly a novel genetic expression system tailored for optimizing biotherapeutics production through optogenetic regulation.

### Background of the Invention

Biopharmaceuticals are a class of medical drugs produced using biotechnology. They are typically made from proteins and/or nucleic acids that are derived from living organisms, including humans, animals, plants, and microorganisms. These drugs are used for therapeutic or diagnostic purposes and include a wide range of products such as:
- Antibodies and antibody derivatives: Targeted therapies for conditions like cancer and autoimmune diseases.
- Vaccines: Preventative treatments for infectious diseases.
- Recombinant proteins: Engineered proteins used to treat diseases like diabetes (e.g., insulin) and growth hormone deficiencies.
- Gene therapies: Treatments that involve modifying a person's genes to treat or cure disease.
- Cell therapies: Using living cells to treat or repair tissues and organs.

In recent years, biopharmaceuticals have demonstrated their potential to provide breakthrough treatments for many life-threatening and chronic conditions, offering more precise and effective therapeutic options compared to traditional small molecule drugs.

Many of these biopharmaceuticals are made in cell culture, either using microbial systems or mammalian cell lines as production host. These cell systems are typically genetically modified to express recombinant genes encoding the genes for the protein or RNA subcomponents of the drug substance. Until now, genetic expression systems for producing proteins, especially biotherapeutics, have been either constitutively expressed or controlled solely through conventional chemical signals, predominantly by the addition of specific chemicals to the culture. For instance, the addition of cumate (4-isopropylbenzoic acid) can induce the production of certain proteins toxic or challenging for cells to express, thereby initiating the biosynthesis of these proteins only once the cell has attained specific desirable metabolic and/or physiological states. However, such control mechanisms are severely limited: the introduction of chemical compounds is irreversible or may require laborious removal to be reversed, the related regulation is inflexible, typically single-channeled (addressing only a single gene per time), and often characterized by limited dynamic range. Moreover, modern biotherapeutics frequently comprise multiple components, such as in the case of bispecific antibodies, consisting of three or more protein chains. Unbalanced quantities or concentrations of subcomponents of the biotherapeutic can lead to increased levels of unintended by-products (e.g., greater amounts of unassembled subcomponents, homomultimers, assemblies with out-of-balance numbers of subcomponents) and reduced yields of the intended product. To ameliorate the unbalanced expression of different genes within a cell, countermeasures have been employed, e.g., through supertransfection (Carver et al., 2020). Nevertheless, such a readjustment of relative or absolute amounts of gene products is entirely static.

Optogenetics represents a relatively novel method for controlling the activity of living cells using light. An example are the light-sensitive systems which provide rapid, noninvasive and reversible control of gene expression upon light exposure (Shimizu-Sato et al., 2002; Pastrana, 2011). As a consequence, light-activated enzymes and transcription factors allow precise control of the biochemical pathways at the level of individual cells. While the temporal response to biochemicals at the genetic level is rather slow, as the biochemical processes take hours or days to be effective, optogenetic methods operate on a millisecond timescale, thus allowing to control gene expression not only in real-time.

### Prior art

US 2021/0147855 A1 discloses optogenetic circuits for controlling protein production with light in *Escherichia coli.* Using these circuits, gene expression can be induced in darkness and repressed under blue light. The disclosed system thus uses light as a suitable alternative to chemical induction for microbial production of chemicals and proteins. In particular, a system is provided, containing (1) a first sequence encoding a first repressor, under a first promoter, the first promoter being a light-controllable promoter, (2) a second sequence encoding a second repressor, under a second promoter, the second promoter being controllable by the first repressor, and (3) a third sequence encoding a gene of interest under a third promoter, the third promoter being controllable by the second repressor.

WO 2013/074911 A1 discloses a system to control gene expression with blue light, in particular methods for light-dependent gene regulation using a light-responsive DNA-binding protein. The system includes the photosensitive protein EL222 (E. *litoralis* 222) containing a LOV-domain as well as a DNA binding domain (DBD) and being fused both with one nuclear localization signal (NLS) and one activation domain. The system further includes a recombinant nucleic acid molecule containing five EL222 DNA binding sites fused to a minimal promoter and allowing moderate to strong blue light induced transcription. The EL222 protein is modified for use in eukaryotic cells by adding a nuclear localization sequence (NLS) and a transcriptional activation domain (VP16-AD) from the herpes simplex virus VP 16 protein. The DNA binding sites are assembled in tandem repeats upstream of a minimal promoter. In the dark, the VP16-EL222 chimera cannot activate a luciferase reporter construct under the control of five-tandem copies of EL222 DNA binding sites (EL222-DBS), while exposure to blue light activates the VP16-EL222 protein allowing it to turn on luciferase transcription.

Accordingly, optogenetics represents a method for controlling numerous processes within living cells through the genetic integration of photoreceptors (e.g., Rost et al., 2017; Hansen et al, 2020). By coupling photoreceptors with effector proteins such as enzymes, protein dimerization domains, ion channels, transcription factors, or signal transduction factors different processes within cells can be governed using light.

### Summary of the invention

It is the object of the invention to provide a method and expression system for producing complex biologically active molecules, in particular complex biopharmaceuticals/biotherapeutics, enabling full flexibility in process control as well as real-time regulation of the entire bioproduction process.

The object is met by providing a method for producing at least one biologically active molecule, in particular a biotherapeutic, by optogenetic control of gene expression in at least one eukaryotic or prokaryotic cell, wherein the biologically active molecule comprises at least one protein of interest comprising at least two different subunits, and wherein the method comprises:
- introducing into the cell with at least one first expression cassette comprising at least one first nucleic acid sequence linked to a first promoter, wherein the first nucleic acid sequence encodes at least one photosensitive transcription factor,
- introducing into the cell with a second expression cassette comprising at least one second nucleic acid sequence encoding a first of the at least two subunits of the protein of interest and being operably linked to a second promoter, the second promoter being operably linked to at least one transcription factor binding site, and
- introducing into the cell a third expression cassette comprising at least one third nucleic acid sequence encoding at least a second of the at least two subunits of the protein of interest and being operably linked to a third promoter,
wherein expression of the second nucleic acid sequence is controlled through activation or repression of the second promoter by illuminating the cell with light comprising at least one wavelength that triggers at least one structural change of the photosensitive transcription factor.

Since illumination of the cells can be controlled through a software controlling a hardware for generating or regulating light, the method according to the invention thus may use, for example, an expression system comprising control interfaces operating bio-digitally instead of purely chemically-analog, particularly a polynucleotide encoding a transcription factor under the control of a promoter, alongside an optimized light-activatable protein-encoding polynucleotide that modulates promoter activity in response to light stimuli. This innovative approach enables dynamic modulation of gene expression, addressing key challenges in biotherapeutics manufacturing. The genetic expression system and associated methodologies used in the method according to the invention represent a significant advancement in biotherapeutics production, offering unparalleled control and optimization opportunities for the development of complex next-generation biopharmaceuticals. That is, the method according to the invention enables full flexibility in process control as well as real-time process regulation of the entire production of complex biopharmaceuticals, so that it is possible to balance or individually temporally dose/pulse multiple components (subunits) within complex protein products, enhancing product quality and therapeutic efficacy. Basically, the application of optogenetics to biopharmaceutical production not only allows to manipulate bioprocesses with unparalleled temporal precision and favorable side effect profiles but also offers light sensors introduced into cells via genetical manipulation as novel control elements, providing the opportunity to establish unique bio-physical interfaces for more automated bioproduction of the future. Additionally, feeding analytical data recorded during the cell culture process as data inputs into the control system of an illumination apparatus executing illumination programs used to temporally control intracellular processes permit the establishment of closed real-time bioprocess control loops as well as seamless integration of advanced data processing methods like machine learning/systems for artificial intelligence into future, fully controlled bioproduction environments, e.g. to automatically avoid process deviations and achieve optimal output.

It is a particular benefit of the method according to the invention that expression/secretion of a protein of interest comprising at least two different subunits can be regulated by the presence and relative concentrations and/or temporally controlled expression of different amounts of at least one light-dependently expressed subunit. For example, limiting expression of a light-dependent subunit restrains the production of the assembled protein of interest, while enhancing expression of a light-dependent subunit promotes the production of the assembled protein of interest. Thus, expression/secretion of complex biologically active molecules can be reliably and precisely regulated by different combination of light-dependent subunits of a protein or protein-nucleic acid complex of interest and the expression of the protein or protein-nucleic acid complex of interest and by-products can be influenced by the relative amounts and/or timing of the subunits expressed. Exact balancing and/or timing of expression of the subunits of a product (protein or protein-nucleic acid complex of interest) has a striking effect on the formation of intended product vs by-products, their relative concentrations, and thus on unpurified product quality, which has an impact on necessary purification efforts, yields of purified product and thus in sum production costs per unit of the product.

"Structural change" as used herein in the sense of the invention refers to any change in the photosensitive transcription factor's conformation and/or secondary or tertiary structure that causes, for example, an exposition of a formerly hidden domain, a dimerization, a multimerization, or the like. For example, the light induction may cause di-/multimerization of a photosensitive transcription factor, whereas said di-/multimerization is a prerequisite to either bind to a transcription factor binding site or to another factor that activates gene expression.

In an advantageous embodiment of the invention, the structural change causes binding or release of the photosensitive transcription factor to/from the transcription factor binding site. For example, if the photosensitive transcription factor binds to the transcription factor binding site when the cell is illuminated using a light source emitting at least one wavelength that triggers this binding, the gene expression controlling domain can control expression of the second nucleic acid sequence encoding a first of the at least two subunits of the protein of interest. That is, expression of the second nucleic acid sequence can be directly (i.e. in real-time) controlled through light dose-dependent activation (upregulation) or repression (downregulation) of the second promoter by switching on a suitable light source. Vice versa, if the light triggers release of the photosensitive transcription factor from the transcription factor binding site, the activating or repressing effect of the gene expression controlling domain can be promptly terminated by switching the light source on. The third nucleic acid sequence encoding the at least second of the at least two subunits of the protein of interest is expressed under the control of the third promoter which may be a constitutive promoter. Since light dose or pulsing can be used to modulate the stimulus, regulation of the production of a complex protein of interest comprising at least two subunits can thus be controlled in real-time, e.g. by balancing or temporally controlling at least the expression of the second nucleic acid sequence. However, it is also possible to control expression of the third (and/or further) nucleic acid sequence(s) through illumination by using a third (and/or further) promoter being operably linked to at least one photosensitive or chemically controlled transcription factor binding site. In such an embodiment of the invention, additional fine-tuning of the production process can be achieved.

In another advantageous embodiment of the invention, the structural change causes nuclear trafficking, i.e. nuclear import and/or export, of the photosensitive transcription factor. Accordingly, the photosensitive transcription factor can bind to a promoter or to another factor binding to a promotor to activate or repress gene expression (e.g., expression of the second nucleic acid) when imported into the nucleus, or reduce transcriptional activation or repression when exported from the nucleus.

The biologically active molecules may be, for example, blood factors, thrombolytic agents, hormones such as insulin or growth hormones, cytokines, peptides, receptors, chaperons, structural proteins, motor proteins, signaling peptides or proteins, adhesion proteins, defense or transport proteins, protease inhibitors, toxins or venoms, vaccines, viral vectors, virus-like particles, therapeutic enzymes, antibodies, nanobodies or protein-nucleic acid complexes, hematopoietic growth factors such as erythropoietin, interferons, interleukins, chimeric antigen receptors, protein nanoparticles, or protein scaffolds. Basically, the biologically active moleculecan be any biopharmaceutical or biotherapeutic, i.e. any pharmaceutical drug product manufactured in, extracted from, and/or semisynthesized by biological sources such as living cells, cell extracts, cell components, and/or artificial biochemical compounds or systems. In an advantageous approach, the method according to the invention can further be employed for the development of a stably transfected cell line optimized for consistent biopharmaceutical production, ensuring reliability and scalability in manufacturing processes.

In a further advantageous embodiment of the invention, the photosensitive transcription factor comprises at least one gene expression controlling domain and/or forms a complex with at least one gene expression controlling domain, wherein the gene expression controlling domain comprises a transcriptional activation domain or a transcriptional repression domain. For example, if the photosensitive transcription factor binds to the transcription factor binding site when the cell is illuminated using a light source emitting at least one wavelength that triggers this binding, the gene expression controlling domain can control expression of the second nucleic acid sequence encoding a first of the at least two subunits of the protein of interest. To this end, the gene expression controlling domain may comprise a transcriptional activation domain, such as VP16, for activating the second promoter, or a transcriptional repression domain, such as KRAB, for repressing the second promoter. In an advantageous embodiment of the invention, the transcriptional activation domain is selected from the group consisting of VP16, VP64, p65, RTA, and any combination thereof. In a preferred embodiment, the transcriptional activation domain comprises VP64 as four repetitions of one region of the herpes simplex virus VP16 transcription activation domain (TAD), each separated by one Glycine-Serine (GS) linker, p65 as a fragment of the human RelA TAD, and RTA as the transcriptional activation domain from the human Epstein-Barr virus. Additionally, VP64 and p65 sequences are separated by an alanine-containing linker, while p65 and RTA are separated by a triple GS-linker. The combination of these activation domains results in significantly enhanced activation of the second promoter.

In another advantageous embodiment of the invention, the first promoter is a constitutive promoter, or the first promoter is operably linked to at least one regulatory sequence. If the first promoter is a constitutive promoter such as the cytomegalovirus (CMV) immediate-early promoter, RNA Pol III promoter H1 or phosphoglycerate kinase 1 (PGK) promoter, the photosensitive transcription factor is continually expressed without the need to induce the expression. The first expression cassette thus represents a non-regulated photoswitch providing a constant level of photosensitive molecules within the cell sufficient to activate or repress at least one of the second or the third expression cassette. In this case, the amount of the photosensitive transcription factor within the cell is always high, so that the transcription factor level in its active state induced by illumination with a light source is not a limiting factor for the production of the protein of interest. Alternatively, if the first promoter is operably linked to at least one regulatory sequence, expression of the photosensitive transcription factor can be controlled, for example, to avoid unnecessary expression thereof. In the latter embodiment, the regulatory sequence may comprise a transcription factor binding site, wherein the first promoter and thus expression of the first nucleic acid sequence encoding the photosensitive transcription factor is induced or stimulated (upregulated) by illuminating the cell with light comprising at least one wavelength that triggers binding of the photosensitive transcription factor to a transcription factor binding site of the regulatory sequence. In this advantageous embodiment of the invention, the first expression cassette represents a self-stimulating photoswitch providing a high number of photosensitive transcription factor molecules within the cell only upon illumination. In this embodiment a lower number of photosensitive transcription factor molecules is expressed in the dark through basal activity of the first promoter that is sufficient to initiate higher expression of the gene encoding itself upon commencing illumination. By limiting the expression of the photosensitive transcription factor, possible cytotoxic effects or metabolic burden of high-level expression can be eliminated, or at least reduced and higher dynamic ranges can be achieved though light-dependent signal amplification via self-regulation of the photosensitive transcription factor.

In another advantageous embodiment of the invention, the third promoter is a constitutive promoter. If the third promoter is a constitutive promoter, the third nucleic acid sequence is continually expressed without the need to induce the expression. In this embodiment, expression of the third nucleic acid is not controlled and thus fine-tuning of the production (assembly) of the protein of interest is essentially accomplished by controlling the expression of the second nucleic acid. Alternatively, the third promoter can be operably linked to at least one transcription factor binding site, wherein expression of the third nucleic acid sequence is controlled through activation or repression of the third promoter by illuminating the cell with light comprising at least one wavelength that triggers at least one structural change of said photosensitive transcription factor or another photosensitive transcription factor. Preferably, the photosensitive transcription factor is the one encoded by the first nucleic acid sequence. But optionally, it might also be advantageous in certain applications that a different photosensitive transcription factor is provided in order to control the third promoter. That is, the further promoter sequence might be controlled by the same or another independent photosensitive transcription factor, e.g., sensitive to another wavelength of light.

The structural change may cause binding or release of the photosensitive transcription factor to/from the transcription factor binding site, or binding or release between at least one DNA binding subunit and at least one transcription activating subunit of the transcription factor. Accordingly, if light triggers the binding of a photosensitive transcription factor to the transcription factor binding site, expression of the third nucleic acid sequence can be controlled in real-time through activation (upregulation) or repression (downregulation) of the third promoter by switching-on a suitable light source. If light triggers the release of a photosensitive transcription factor from the transcription factor binding site, switching-on the light source promptly terminates the activating or repressing effect of the gene expression controlling domain. Since light dose or pulsing schemes can be used to modulate the stimulus, regulation of the production of a complex protein of interest comprising at least two subunits can thus be controlled in real-time by balancing or temporally dose/pulse the expression of both the second and the third nucleic acid sequence. In this advantageous embodiment of the invention, additional fine-tuning of the production process can be achieved.

The structural change may also cause nuclear trafficking, i.e. nuclear import and/or export, of the photosensitive transcription factor. Accordingly, the photosensitive transcription factor can bind to a promoter or to another factor binding to a promotor to activate or repress expression of the third nucleic acid sequence when imported into the nucleus, or reduce transcriptional activation or repression when exported from the nucleus.

For example, if the protein of interest is an antibody, one of the at least two subunits of the protein of interest may be a light chain of the antibody and another one of the at least two subunits of the protein of interest may be a heavy chain or a fusion of a light and a heavy chain of the antibody. That is, for example, the first of the at least two subunits of the protein of interest may be a light chain of the antibody and the second of the at least two subunits of the protein of interest may be a heavy chain of the antibody. Vice versa, the first of the at least two subunits of the protein of interest may be a heavy chain of the antibody and the second of the at least two subunits of the protein of interest may be a light chain of the antibody. Consequently, the method according to the invention allows for optogenetic control of monoclonal antibody synthesis, offering tailored production strategies and enhanced antibody engineering capabilities.

In another advantageous embodiment of the invention, at least one additional expression cassette is introduced into the cell, the additional expression cassette comprising at least one further nucleic acid sequence encoding at least one further subunit of the at least two subunits of the protein of interest and being operably linked to a further promoter sequence. That is, it is also possible to control expression of any further nucleic acid sequence and thus further subunits of the protein of interest through illumination by using at least one further promoter being operably linked to at least one transcription factor binding site. In such an embodiment of the invention, additional fine-tuning of the entire production process can be achieved. Moreover, full flexibility in process control as well as real-time process regulation of the production of complex biopharmaceuticals is enabled, so that it is possible to balance multiple subunits within complex protein products, enhancing product quality and therapeutic efficacy.

In this embodiment, the further promoter sequence may comprise a constitutive promoter. If the further promoter is constitutive, the third nucleic acid sequence is continually expressed without the need to induce the expression. In this embodiment, the expression of each further nucleic acid is not controlled and thus fine-tuning of the entire production (assembly) of the protein of interest which may comprise several subunits is essentially accomplished by controlling the expression of the second, and optionally the third, nucleic acid. Alternatively, at least one of the further promoter sequences can be operably linked to at least one transcription factor binding site, wherein expression of the further nucleic acid sequence is controlled through activation or repression of the further promoter sequence by illuminating the cell with light comprising at least one wavelength that triggers at least one structural change of said photosensitive transcription factor or another photosensitive transcription factor. Preferably, the photosensitive transcription factor is the one encoded by the first nucleic acid sequence. But optionally, it might also be advantageous in certain applications that a different photosensitive transcription factor is provided in order to control the further promoter sequence. That is, the further promoter sequence might be controlled by the same or another independent photosensitive transcription factor, e.g., sensitive to another wavelength of light.

The structural change may cause binding or release of the photosensitive transcription factor to/from the transcription factor binding site, or binding or release between at least one DNA binding subunit and at least one transcription activating subunit of the transcription factor. Accordingly, if light triggers the binding of a photosensitive transcription factor to the transcription factor binding site, expression of at least one of the further nucleic acid sequences can be controlled in real-time through activation (upregulation) or repression (downregulation) of the further promoter by switching on a suitable light source.

If the light triggers release of the photosensitive transcription factor from the transcription factor binding site, switching-on the light source promptly terminates the activating or repressing effect of the gene expression controlling domain. Regulation of the production of complex proteins of interest comprising several subunits can thus be controlled in real-time by balancing the expression of the second, optionally the third, and at least one of the further nucleic acid sequences. In this advantageous embodiment of the invention, additional fine-tuning of the entire production process can be achieved.

The structural change may also cause nuclear trafficking, i.e. nuclear import and/or export, of the photosensitive transcription factor. Accordingly, the photosensitive transcription factor can bind to a promoter or to another factor binding to a promotor to activate or repress expression of the further nucleic acid sequence(s) when imported into the nucleus, or reduce transcriptional activation or repression when exported from the nucleus.

For example, the protein of interest may be a bispecific antibody, wherein one of the at least two subunits of the protein of interest may be a light chain of the antibody, another one of the at least two subunits of the protein of interest may be a heavy chain of the antibody, and at least one further subunit of the at least two subunits of the protein of interest may be a single chain variable fragment (scFv), an antigen binding fragment (Fab), or a fusion of a light and a heavy chain of the antibody. For example, the first of at least two subunits might be a first light chain, the second of the at least two subunits a first heavy chain, the third of the at least two subunits a second light chain and the fourth of the at least two subunits a second heavy chain. This option includes the production of two different heavy chains and two different light chains (w/o hybrid). Moreover, the method according to the invention might also be used to produce, for example, at least two different single chain variable fragments (= hybrid chains) together (w/o light chain or heavy chain). The method according to the invention can thus modulate bispecific antibody production, enabling the improved production of advanced therapeutic molecules with multiple binding specificities.

According to the invention, all expression cassettes encoding a subunit of the protein of interest can be controlled by the same photosensitive transcription factor. Alternatively, different expression cassettes can be controlled by different photosensitive transcription factors, or at least one of the expression cassettes can be controlled by a different photosensitive transcription factor while the other expression cassettes are controlled by the same photosensitive transcription factor, and so on.

For example, the wavelength of the light may be selected from a wavelength between 400 nm and 500 nm (blue light) or between 640 and 780 nm (red and far-red light).

Basically, the optogenetic method according to the invention can be combined with other methods, for example, with chemical control methods, especially for the production of different subcomponents of the biologically active molecule.

The object is further met by providing an expression system for producing at least one biologically active molecule, in particular a biotherapeutic, by optogenetic control of gene expression in at least one eukaryotic or prokaryotic cell, wherein the biologically active molecule comprises at least one protein of interest comprising at least two subunits, said expression system comprising:
- at least one first expression cassette comprising at least one first nucleic acid sequence linked to a first promoter, wherein the first nucleic acid sequence encodes at least one photosensitive transcription factor comprising a gene expression controlling domain and/or having a structure that enables forming a complex with at least one gene expression controlling domain,
- a second expression cassette comprising at least one second nucleic acid sequence encoding a first of the at least two subunits of the protein of interest and being operably linked to a second promoter, wherein the second promoter is operably linked to at least one transcription factor binding site and activatable or repressible by illumination with light comprising at least one wavelength that triggers at least one structural change of the photosensitive transcription factor, and
- a third expression cassette comprising at least one third nucleic acid sequence encoding at least a second of the at least two subunits of the protein of interest and being operably linked to a third promoter.

For example, since illumination of the cells can be controlled through a software controlling a hardware for generating or regulating light, the expression system according to the invention may comprise control interfaces operating bio-digitally instead of purely chemically-analog, in particular at least one polynucleotide encoding at least one transcription factor under the control of at least one promoter, alongside at least one light-activatable protein-encoding polynucleotide that modulates promoter activity in response to light stimuli. This innovative approach enables dynamic modulation of gene expression, addressing key challenges in biotherapeutics manufacturing. The expression system according to the invention thus represents a significant advancement in biotherapeutics production, offering unparalleled control and optimization opportunities for the development of complex next-generation biopharmaceuticals. That is, the expression system according to the invention enables full flexibility in process control as well as real-time process regulation of the entire production of complex biopharmaceuticals, so that it is possible to balance and/or individually temporally dose/pulse multiple components (subunits) within complex protein products, enhancing product quality and thus purity of the product before purification and/or production efficiency by reducing by-products that are either inactive or show unintended activities. Reducing by-products also leads to increased therapeutic efficacy, since more effective molecules are provided with the same amount of protein. In turn, if smaller relative amounts of by-products need to be separated from the intended protein or protein-nucleic acid complex of interest, subsequent purification steps might be simplified or omitted, resulting in time and/or material savings as well as reduced loss of the intended protein or protein-nucleic acid complex.

It is a particular benefit of the expression system according to the invention that expression/secretion of a protein of interest comprising at least two different subunits can be regulated by the presence and relative concentrations and/or temporally controlled expression of different amounts of at least one light-dependently expressed subunit. For example, limiting expression of a light-dependent subunit restrains the production of the assembled protein of interest, while enhancing expression of a light-dependent subunit promotes the production of the assembled protein of interest. Thus, expression/secretion of complex biologically active molecules can be reliably and precisely regulated by different combination of light-dependent subunits of a protein of interest and the expression of the protein of interest and by-products can be influenced by the relative amounts and/or timing of the subunits expressed. Exact balancing and/or timing of expression of the subunits of a product (protein of interest) has a striking effect on the formation of intended product vs by-products, their relative concentrations, and thus on unpurified product quality, which has an impact on necessary purification efforts, yields of purified product and thus in sum production costs per unit of the product.

"Structural change" as used herein in the sense of the invention refers to any change in the photosensitive transcription factor's conformation and/or tertiary structure that causes, for example, an exposition of a formerly hidden domain, a dimerization, a multimerization, or the like. For example, the light induction may cause di-/multimerization of a photosensitive transcription factor, whereas said di-/multimerization is a prerequisite to either bind to a transcription factor binding site or binding to another factor that activates gene expression.

The structural change may cause, for example, binding or release of the photosensitive transcription factor to/from the transcription factor binding site. For example, if the photosensitive transcription factor binds to the transcription factor binding site when the cell is illuminated using a light source emitting at least one wavelength that triggers this binding, the gene expression controlling domain can control expression of the second nucleic acid sequence encoding a first of the at least two subunits of the protein of interest. That is, expression of the second nucleic acid sequence can be directly (i.e. in real-time) controlled through light dose-dependent activation (upregulation) or repression (downregulation) of the second promoter by switching on a suitable light source. Vice versa, if the light triggers release of the photosensitive transcription factor from the transcription factor binding site, the activating or repressing effect of the gene expression controlling domain can be promptly terminated by switching the light source on. The third nucleic acid sequence encoding the at least second of the at least two subunits of the protein of interest is expressed under the control of the third promoter which may be a constitutive promoter. Since light dose or pulsing can be used to modulate the stimulus, regulation of the production of a complex protein of interest comprising at least two subunits can thus be controlled in real-time by balancing at least the expression of the second nucleic acid sequence. However, it is also possible to control expression of the third (and/or further) nucleic acid sequence(s) through illumination by using a third (and/or further) promoter being operably linked to at least one photosensitive or chemically controlled transcription factor binding site. In such an embodiment of the invention, additional fine-tuning of the production process can be achieved.

The structural change may further cause, alternatively or additionally, nuclear trafficking, i.e. nuclear import and/or export, of the photosensitive transcription factor. Accordingly, the photosensitive transcription factor can bind to a promoter or to another factor binding to a promoter to activate or repress gene expression only when located in the nucleus, which in turn can be controlled by exposure to light.

The expression system according to the invention may further comprise, for example, at least one artificial polynucleotide designed for fine-tuning of the expression of complex biotherapeutic products, allowing for precise control over product characteristics and performance. The expression system according to the invention may also comprise, for example, at least one artificial polynucleotide encoding a light-activatable (photo-sensitive) transcription factor efficiently activating a specific promoter in living cells such as, for example, mammalian, insect, bacterial, fungal or plant cells. Moreover, the expression system according to the invention may comprise at least one artificial polynucleotide encoding a red or blue light activated transcription factor efficiently activating a specific promoter in mammalian cells.

The biologically active molecules may be, for example, blood factors, thrombolytic agents, hormones such as insulin or growth hormones, cytokines, peptides, receptors, chaperons, structural proteins, motor proteins, signaling peptides or proteins, adhesion proteins, defense or transport proteins, protease inhibitors, toxins or venoms, nanobodies or protein-nucleic acid complexes, hematopoietic growth factors such as erythropoietin, interferons, interleukins as well as vaccines, therapeutic enzymes, chimeric antigen receptors, protein nanoparticles, protein scaffolds, virus-like particles, viral vectors, or antibodies. Basically, the biologically active molecule can be any biopharmaceutical and/or biotherapeutic, i.e. any pharmaceutical drug product manufactured in, extracted from, and/or semi synthesized by biological sources such as living cells, cell extracts, cell components, and/or artificial biochemical compounds or systems. In an advantageous approach, the method according to the invention can further be employed for the development of a stably transfected cell line optimized for consistent biopharmaceutical production, ensuring reliability and scalability in manufacturing processes.

In an advantageous embodiment of the invention, the expression system further comprises additional expression cassettes, each additional expression cassette comprising at least one further nucleic acid sequence encoding at least one further subunit of the at least two subunits of the protein of interest and being operably linked to a further promoter sequence. That is, by providing additional expression cassettes, controlling the expression of further nucleic acid sequences and thus further subunits of the protein of interest through illumination can be ensured, preferably by using at least one further promoter being operably linked to at least one transcription factor binding site. This allows for precise and effective production of complex biopharmaceuticals, wherein it is possible to balance or temporally dose/pulse multiple subunits within complex protein products, enhancing product quality and thus purity of the product before purification and/or production efficiency by reducing by-products that are either inactive or show unintended activities. Reducing by-products also leads to increased therapeutic efficacy, since more effective molecules are provided with the same amount of protein. In turn, if smaller relative amounts of by-products need to be separated from the intended protein or protein-nucleic acid complex of interest, subsequent purification steps might be simplified or omitted, resulting in time and/or material savings as well as reduced loss of the intended protein or protein-nucleic acid complex.

Each promotor / promoter sequence of the expression system according to the invention may comprise a constitutive promoter or may be operably linked to at least one transcription factor binding site, wherein expression of the nucleic acid sequence (gene) regulated by the promoter is controlled through activation or repression of the promoter sequence by illuminating the cell with light comprising at least one wavelength that triggers at least one structural change of a photosensitive transcription factor.

In another advantageous embodiment of the invention, the photosensitive transcription factor may comprise at least one light-oxygen-voltage-sensing domain (LOV domain), preferably EL222, or at least one cryptochrome (CRY), or at least one phytochrome, preferably phytochrome B. Alternatively or additionally, the transcription factor binding site comprises at least binding site selected from the group consisting of at least one (C120), GAL4, TetR, lexA, and lacR binding site.

In a further advantageous embodiment of the invention, the gene expression controlling domain of the photosensitive transcription factor may comprise at least one transcriptional activation domain, preferably selected from the group consisting of VP16, VP64, p65, RTA, and any combination thereof, or a transcriptional repression domain, preferably KRAB. The photosensitive transcription factor may further comprise at least one nuclear localization signal (NLS) and/or at least one DNA-binding domain (DBD). An advantageous combination of transcriptional activation domains is VP64-p65-RTA, preferably operably linked to EL222. This combination surprisingly provides a significantly enhanced, very effective activation of the second promoter, in particular of an inducible minimum promoter.

The object is also met by providing a kit or composition for producing at least one biologically active molecule, in particular a biotherapeutic, wherein the biologically active molecule comprises at least one protein of interest comprising at least two subunits, by optogenetic control of gene expression of at least one of the at least two subunits in at least one eukaryotic or prokaryotic cell, comprising the expression system as described above.

The object is further met by providing a photosensitive transcription factor comprising the light-oxygen-voltage-sensing domain EL222, a transcriptional activation domain comprising VP64, p65, and RTA, and two nuclear localization signals. Preferably, the photosensitive transcription factor comprises the amino acid sequence according to SEQ ID NO: 1 set forth hereunder. 
PKKKRKVDALDDFDLDMLGSDALDDFDLDMLGSDALDDFDLDMLGSDALDDF
DLDMLAAAEFQYLPDTDDRHRIEEKRKRTYETFKSIMKKSPFSGPTDPRPPPR
RIAVPSRSSASVPKPAPQPYPFTSSLSTINYDEFPTMVFPSGQISQASALAPAP
PQVLPQAPAPAPAPAMVSALAQAPAPVPVLAPGPPQAVAPPAPKPTQAGEGT
LSEALLQLQFDDEDLGALLGNSTDPAVFTDLASVDNSEFQQLLNQGIPVAPHT
TEPMLMEYPEAITRLVTGAQRPPDPAPAPLGAPGLPNGLLSGDEDFSSIADMD
FSALLSQISSGSGSGSRDSREGMFLPKPEAGSAISDVFEGREVCQPKRIRPFH
PPGSPWANRPLPASLAPTPTGPVHEPVGSLTPAPVPQPLDPAPAVTPEASHL
LEDPDEETSQAVKALREMADTVIPQKEEAAICGQMDLSHPPPRGHLDELTTTL
ESMTEDLNLDSPLTPELNEILDTFLNDECLLHAMHISTGLSIFDTSLFEFGADDT
RVEVQPPAQWVLDLIEASPIASVVSDPRLADNPLIAINQAFTDLTGYSEEECVG
RNCRFLAGSGTEPWLTDKIRQGVREHKPVLVEILNYKKDGTPFRNAVLVAPIY
DDDDELLYFLGSQVEVDDDQPNMGMARRERAAEMLRTLSPRQLEVTTLVAS
GLRNKEVAARLGLSEKTVKMHRGLVMEKLNLKTSADLVRIAVEAGIEFSASKR
PAATKKAGQAKKKK (SEQ ID NO: 1)
 **SEQ ID NO: 1** consists of the following sub-sequences:
PKKKRKV (**SV40 NLS, SEQ ID NO: 2)**
DALDDFDLDMLGSDALDDFDLDMLGSDALDDFDLDMLGSDALDDFDLDML **(VP64, SEQ ID NO: 3),**
AAA **(Linker, SEQ ID NO: 4),** 
EFQYLPDTDDRHRIEEKRKRTYETFKSIMKKSPFSGPTDPRPPPRRIAVPSRS
SASVPKPAPQPYPFTSSLSTINYDEFPTMVFPSGQISQASALAPAPPQVLPQA
 
PAPAPAPAMVSALAQAPAPVPVLAPGPPQAVAPPAPKPTQAGEGTLSEALLQ
LQFDDEDLGALLGNSTDPAVFTDLASVDNSEFQQLLNQGIPVAPHTTEPMLME
YPEAITRLVTGAQRPPDPAPAPLGAPGLPNGLLSGDEDFSSIADMDFSALLSQI
SS (RelA (p65) AD, SEQ ID NO: 5),
GSGSGS **(Linker, SEQ ID NO: 6),** 
RDSREGMFLPKPEAGSAISDVFEGREVCQPKRIRPFHPPGSPWANRPLPASL
APTPTGPVHEPVGSLTPAPVPQPLDPAPAVTPEASHLLEDPDEETSQAVKALR
EMADTVIPQKEEAAICGQMDLSHPPPRGHLDELTTTLESMTEDLNLDSPLTPE
LNEILDTFLNDECLLHAMHISTGLSIFDTSLF (Rta AD, SEQ ID NO: 7),
 
PKKKRKVDALDDFDLDMLGSDALDDFDLDMLGSDALDDFDLDMLGSDALDDF
DLDMLAAAEFQYLPDTDDRHRIEEKRKRTYETFKSIMKKSPFSGPTDPRPPPR
RIAVPSRSSASVPKAPAPQPYPFTSSLSTINYDEFPTMVFPSGQISQASALAPAP
PQVLPQAPAPAPAPAMVSALAQAPAPVPVLAPGPPQAVAPPAPKPTQAGEGT
LSEALLQLQFDDEDLGALLGNSTDPAVFTDLASVDNSEFQQLLNQGIPVAPHT
TEPMLMEYPEAITRLVTGAQRPPDPAPAPLGAPGLPNGLLSGDEDFSSIADMD
FSALLSQISSGSGSGSRDSREGMFLPKPEAGSAISDVFEGREVCQPKRIRPFH
PPGSPWANRPLPASLAPTPTGPVHEPVGSLTPAPVPQPLDPAPAVTPEASHL
LEDPDEETSQAVKALREMADTVIPQKEEAAICGQMDLSHPPPRGHLDELTTTL
ESMTEDLNLDSPLTPELNEILDTFLNDECLLHAMHISTGLSIFDTSLFEFGADDT
RVEVQPPAQWVLDLIEASPIASVVSDPRLADNPLIAINQAFTDLTGYSEEECVG
RNCRFLAGSGTEPWLTDKIRQGVREHKPVLVEILNYKKDGTPFRNAVLVAPIY
DDDDELLYFLGSQVEVDDDQPNMGMARRERAAEMLRTLSPRQLEVTTLVAS
GLRNKEVAARLGLSEKTVKMHRGLVMEKLNLKTSADLVRIAVEAGI (EL222,
SEQ ID NO: 8),
EFSAS **(Linker, SEQ ID NO: 9**), and
KRPAATKKAGQAKKKK **(Nucleoplasmin NLS, SEQ ID NO: 10)**

The object is further met by providing a nucleic acid molecule comprising a nucleotide sequence encoding the photosensitive transcription factor as described above.

Another aspect of the invention is the provision of a method for producing at least one biologically active molecule, in particular a biotherapeutic, by optogenetic control of gene expression in at least one eukaryotic or prokaryotic cell, wherein the biologically active molecule comprises at least one protein of interest comprising at least two different subunits, wherein the method comprises:
- introducing into the cell at least one first expression cassette comprising at least one first nucleic acid sequence linked to a first promoter, wherein the first nucleic acid sequence encodes at least one photosensitive transcription factor, and
- introducing into the cell a second expression cassette comprising at least one second nucleic acid sequence encoding a first of the at least two subunits of the protein of interest and being operably linked to a second promoter, the second promoter being operably linked to at least one transcription factor binding site, wherein expression of the second nucleic acid sequence is controlled through activation or repression of the second promoter by illuminating the cell with light comprising at least one wavelength that triggers at least one structural change of the photosensitive transcription factor,
wherein the first promoter is operably linked to at least one regulatory sequence, the regulatory sequence comprising at least one transcription factor binding site, wherein the first promoter and thus expression of the first nucleic acid sequence encoding the photosensitive transcription factor is induced or stimulated (upregulated) by illuminating the cell with light comprising at least one wavelength that triggers binding of the photosensitive transcription factor to a transcription factor binding site of the regulatory sequence.

As the first promoter is operably linked to at least one regulatory sequence, expression of the photosensitive transcription factor can be controlled, for example, to avoid unnecessary expression thereof. Expression of the first nucleic acid sequence is directly (i.e. in real-time) controlled through activation (upregulation) or repression (downregulation) of the first promoter by switching on a suitable light source. Vice versa, if the light triggers the release of the photosensitive transcription factor from the transcription factor binding site, the activating or repressing effect of the gene expression controlling domain can be promptly terminated by switching the light source on. That is, the first expression cassette represents a self-stimulating photoswitch providing a high number of photosensitive transcription factor molecules within the cell only upon illumination. In the dark, a lower number of photosensitive transcription factor molecules is expressed, which is, however, still sufficient to initiate higher expression of the gene encoding itself upon commencing illumination. By controlling (limiting) the expression of the photosensitive transcription factor, possible cytotoxic effects can be eliminated, or at least reduced and higher dynamic ranges can be ensured though light-dependent signal amplification via self-regulation of the photosensitive transcription factor..

A further aspect of the invention is the provision of an artificial polynucleotide, in particular for use in a method for producing at least one biologically active molecule, in particular a biotherapeutic, by optogenetic control of gene expression in at least one eukaryotic or prokaryotic cell, wherein the biologically active molecule comprises at least one protein of interest comprising at least two different subunits, wherein the artificial polynucleotide comprises
at least one nucleic acid sequence encoding at least one photosensitive transcription factor linked to at least one promoter sequence, the photosensitive transcription factor comprising a gene expression controlling domain, wherein the gene expression controlling domain optionally comprises a transcriptional activation domain or a transcriptional repression domain,
wherein the promoter sequence is operably linked to at least one regulatory sequence comprising a transcription factor binding site, wherein the promoter sequence and thus expression of the nucleic acid sequence encoding the photosensitive transcription factor is induced and/or stimulated (upregulated) by illuminating the cell with light comprising at least one wavelength that triggers binding of the photosensitive transcription factor to the transcription factor binding site of the regulatory sequence.

As the promoter sequence is operably linked to at least one regulatory sequence, expression of the photosensitive transcription factor can be controlled, for example, to avoid unnecessary expression thereof. Expression of the nucleic acid sequence can be directly (i.e. in real-time) controlled through activation (upregulation) or repression (downregulation) of the promoter sequence by switching on a suitable light source. Vice versa, if the light triggers the release of the photosensitive transcription factor from the transcription factor binding site, the activating or repressing effect of the gene expression controlling domain can be promptly terminated by switching the light source on. That is, the first expression cassette represents a self-stimulating photoswitch providing a high number of photosensitive transcription factor molecules within the cell only upon illumination. In the dark, a lower number of photosensitive transcription factor molecules is expressed, which is, however, still sufficient to initiate higher expression of the gene encoding itself upon commencing illumination. By controlling (limiting) the expression of the photosensitive transcription factor, possible cytotoxic effects can be eliminated, or at least reduced and higher dynamic ranges can be ensured though light-dependent signal amplification via self-regulation of the photosensitive transcription factor.

"Introducing into the cell" as used herein in the sense of the invention refers to the transfer of nucleic acid molecules such as DNA and RNA (genetic material) into at least one cell, especially for either transcription into RNA and/or translation into proteins, including but not limited to transformation (transfer of non-viral genetic material into bacteria), transfection (transfer of non-viral genetic material into eukaryotic cells), and transduction (transfer of genetic material into eukaryotic cells using viral vectors).

The invention is further described in detail with reference to the following examples and figures.

### Brief description of the figures

- **Figure 1**: shows a schematic representation of an exemplary embodiment of an expression system according to the invention comprising expression cassettes for optogenetic control of the production of a monoclonal antibody.
- **Figure 2**: shows a schematic representation of an exemplary embodiment of an expression system according to the invention comprising expression cassettes for optogenetic control of the production of a bispecific antibody.
- **Figure 3**: shows a schematic representation of an exemplary embodiment of a self-stimulating photoswitch according to the invention.
- **Figure 4**: shows a schematic representation of an exemplary embodiment of an expression system according to the invention comprising expression cassettes for balancing several components of a complex protein product.
- **Figure 5**: shows a schematic representation of an exemplary embodiment of a method according to the invention using genetically engineered constructs according to Figure 4 for generating a stably transfected cell line for the production of biopharmaceuticals.
- **Figure 6**: shows the result of a Western blot (WB) analysis under reducing conditions demonstrating light intensity-dependent increase in bispecific antibody (bsAb) expression/secretion upon blue light stimulation.
- **Figure 7**: shows bar diagrams demonstrating the performance of a first expression cassette according to the invention (blue-sensitive photoswitch bluePS "DV").
- **Figure 8**: shows bar diagrams demonstrating the results of a comparison of the protein expression levels mediated by an exemplary embodiment of a first expression cassette according to the invention (blue-sensitive photoswitch bluePS "DV") and different constitutive promoters.
- **Figure 9**: shows the results of Western blot (WB) analyses demonstrating light dose- or pulse-dependent differential expression/secretion of subunits of a bispecific antibody (bsAb).
- **Figure 10**: shows the result of a Western blot (WB) analysis under non-reducing conditions demonstrating light intensity-dependent increase in bispecific antibody (bsAb) expression/secretion upon blue light stimulation.
- **Figure 11**: shows the result of a Western blot (WB) under non-reducing conditions demonstrating that expression/secretion of bispecific antibody (bsAb) complexes is regulated by different amounts of light-dependent chains.

### Detailed description of exemplary and preferred embodiments of the invention

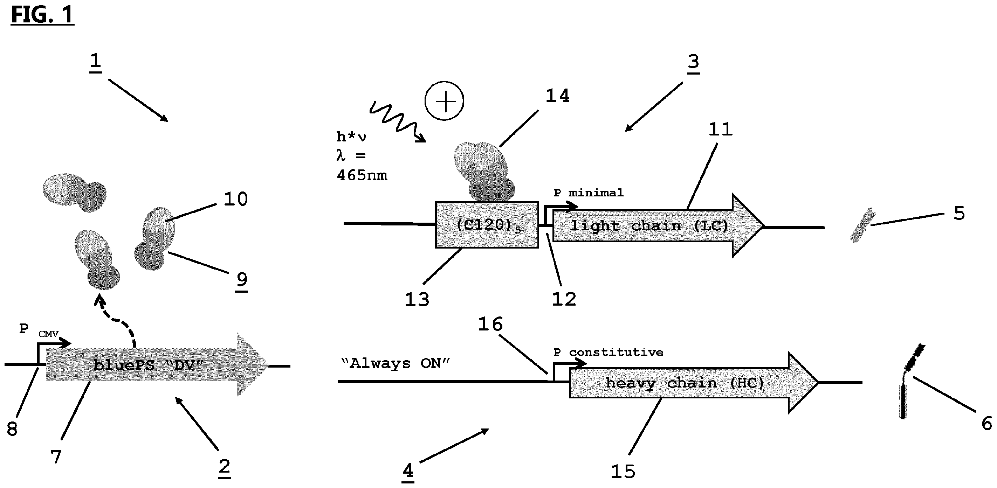

**Figure 1** shows an exemplary embodiment of an expression system 1 comprising three expression cassettes 2, 3, 4 for optogenetic control of the production of a protein of interest such as, in this embodiment, a monoclonal antibody comprising two subunits 5, 6. A first expression cassette 2 comprises a first nucleic acid sequence 7 (" bluePS "DV" ") that is operably linked to a first promoter 8 being disposed upstream of the first nucleic acid sequence 7. The first promoter 8 may be a constitutive promoter such as the cytomegalovirus promoter ("P_{CMV}"), allowing for continual transcription of the first nucleic acid sequence 7. The first nucleic acid sequence 7 encodes a photosensitive transcription factor 9 comprising a gene expression controlling domain 10. The photosensitive transcription factor 9 may comprise at least one light-oxygen-voltage-sensing domain (LOV domain), preferably EL222. The gene expression controlling domain 10 of the photosensitive transcription factor 9 may comprise a transcriptional activation domain such as VP16, VP64, p65, RTA or any combination thereof. The first expression cassette 2 thus represents a constitutively expressed photoswitch providing a constantly sufficient level of photosensitive transcription factors 9 within a cell. For example, the first nucleic acid sequence 7 " bluePS "DV" " may encode a photosensitive transcription factor comprising the light-oxygen-voltage-sensing domain EL222, a transcriptional activation domain comprising VP64, p65, and RTA, and two nuclear localization signals, preferably the amino acid sequence according to SEQ ID NO: 1.

The expression system 1 further comprises a second expression cassette 3 comprising a second nucleic acid sequence 11 encoding the first subunit 5 ("light chain (LC)") of the monoclonal antibody (first gene of interest). The second nucleic acid sequence 11 is operably linked to a second promoter 12 such as an inducible minimal promoter ("Pₘᵢₙᵢₘₐₗ") which is disposed upstream of the second nucleic acid sequence 11. The second promoter 12 is operably linked to a transcription factor binding site 13 ("(C120)s") arranged upstream of the second promoter 12. Illumination of the cell with blue light (λ = 465 nm) triggers dimerization of the photosensitive transcription factors 9 provided by the photoswitch, allowing the resulting dimers 14 to bind to the transcription factor binding site 13. These structural changes (described by Motta-Mena et al., 2014) ensure that the gene expression controlling domain 10 is placed in the vicinity of the second promoter 12 which is, in this embodiment, activated by the transcriptional activation domain. Accordingly, transcription of the second nucleic acid sequence 11 encoding the first subunit 5 ("light chain (LC)") of the monoclonal antibody is rapidly induced by the second promoter 12 upon switching on a suitable light source emitting blue light. If this light source is switched off, the structural changes described above spontaneously reverse in the dark so that binding of the dimer of the transcription factor 14 to the second transcription factor binding site 13 and expression of the second nucleic acid sequence 11 is stopped immediately. Fast and precise optogenetic control of the production of a monoclonal antibody can thus be ensured using the method and expression system according to the invention.

The expression system 1 also comprises a third expression cassette 4 comprising a third nucleic acid sequence 15 encoding the second subunit 6 ("heavy chain (HC)") of the monoclonal antibody (second gene of interest). The third nucleic acid sequence 15 is operably linked to a third promoter 16 being disposed upstream of the third nucleic acid sequence 15. In this embodiment, the third promoter 16 is a constitutive promoter ("P_{constitutive}"), allowing for continual transcription of the third nucleic acid sequence 15. The monoclonal antibody (protein of interest) is then assembled by association of the two subunits 5, 6, wherein production of this complex protein can be precisely balanced and/or its secretion effectively controlled by the optogenetic gene expression system according to the invention, in this embodiment especially by controlling expression of the second nucleic acid sequence 11 (first subunit 5).

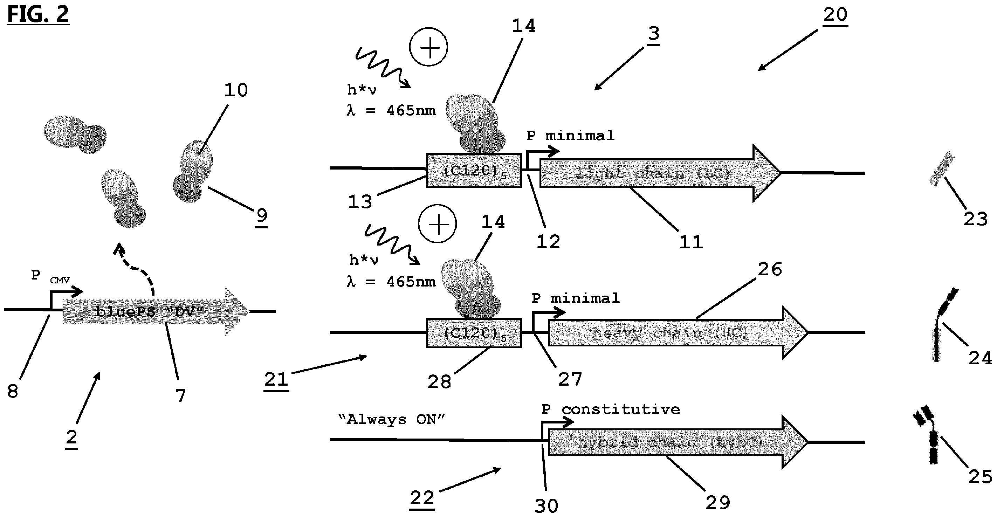

**Figure 2** shows an exemplary embodiment of an expression system 20 comprising four expression cassettes 2, 3, 21, 22 for optogenetic control of the production of a protein of interest such as, in this embodiment, a bispecific antibody comprising three subunits 23, 24, 25. The first and the second expression cassettes 2, 3 are the same as the ones depicted in Figure 1, respectively. Accordingly, the first expression cassette 2 represents a constitutive photoswitch providing a constantly sufficient level of photosensitive transcription factors 9 within a cell and the second expression cassette 3 comprises a second nucleic acid sequence 11 encoding the first subunit 23 ("light chain (LC)") of the bispecific antibody. The first and the second expression cassettes 2, 3 and their respective effects within the cell are described in detail above with reference to Figure 1.

The expression system 20 also comprises a third expression cassette 21 comprising a third nucleic acid sequence 26 encoding the second subunit 24 ("heavy chain (HC)") of the bispecific antibody (second gene of interest). The third nucleic acid sequence 26 is operably linked to a third promoter 27 such as an inducible minimal promoter ("Pₘᵢₙᵢₘₐₗ") which is disposed upstream of the third nucleic acid sequence 26. The third promoter 27 is operably linked to a transcription factor binding site 28 ("(C120)s") arranged upstream of the third promoter 27. Illumination of the cell with blue light (λ = 465 nm) triggers dimerization of the photosensitive transcription factors 9 provided by the photoswitch, allowing the resulting dimers 14 to bind to the transcription factor binding site 28. These structural changes (described by Motta-Mena et al., 2014) ensure that the gene expression controlling domain 10 is placed in the vicinity of the third promoter 27 which is, in this embodiment, activated by the transcriptional activation domain. Accordingly, transcription of the third nucleic acid sequence 26 encoding the second subunit 24 ("heavy chain (HC)") of the bispecific antibody is rapidly induced by the third promoter 27 upon switching on a suitable light source emitting blue light. If this light source is switched off, the structural changes described above spontaneously reverse in the dark so that expression of the third nucleic acid sequence 26 is stopped immediately. Fast and precise optogenetic control of the production of a bispecific antibody can thus be ensured using the method and expression system according to the invention.

The expression system 20 further comprises an additional (fourth) expression cassette 22 comprising a further (fourth) nucleic acid sequence 29 encoding an additional (third) subunit 25 ("hybrid chain (hybC)") of the bispecific antibody (further (third) gene of interest). The fourth nucleic acid sequence 29 is operably linked to a further (fourth) promoter sequence 30 being disposed upstream of the fourth nucleic acid sequence 29. In this embodiment, the fourth promoter 30 is a constitutive promoter ("P_{constitutive}"), allowing for continual transcription of the fourth nucleic acid sequence 29. The bispecific antibody (protein of interest) is then assembled by the association of the three subunits 23, 24, 25, wherein the production of this complex protein can be precisely balanced by the optogenetic gene expression system according to the invention, in this embodiment, especially by controlling expression of the second and the third nucleic acid sequences 11, 26 (first and second subunit 23, 24).

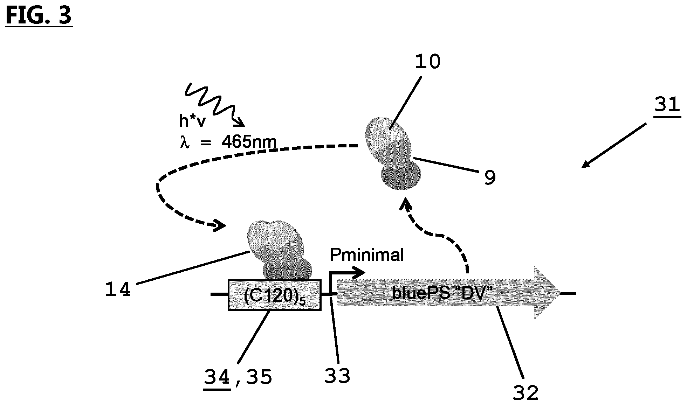

**Figure 3** shows an exemplary embodiment of a self-stimulating photoswitch according to the invention that can be used, for example, in the method according to the invention for optogenetically controlling the production of a protein of interest. The self-stimulating photoswitch comprises an expression cassette 31 comprising a first nucleic acid sequence 32 that is operably linked to a first promoter 33 such as an inducible minimal promoter ("Pₘᵢₙᵢₘₐₗ") being disposed upstream of the first nucleic acid sequence 32. The first nucleic acid sequence 32 encodes a photosensitive transcription factor 9 (" bluePS "DV" ") comprising a gene expression controlling domain 10. The photosensitive transcription factor 9 may comprise at least one light-oxygen-voltage-sensing domain (LOV domain), preferably EL222. The gene expression controlling domain 10 of the photosensitive transcription factor 9 may comprise a transcriptional activation domain such as VP16, VP64, p65, RTA or any combination thereof. In this embodiment, the first promoter 33 is operably linked to at least one regulatory sequence 34 so as to control expression of the photosensitive transcription factor 9, for example, in order to avoid unnecessary expression thereof and increase the dynamic range of either activation or repression of promoters harboring transcription factor binding sites (not shown in this figure) for the photosensitive transcription factor 9. The regulatory sequence 34 comprises a transcription factor binding site 35 ("(C120)₅"), wherein the first promoter 33 and thus the expression of the first nucleic acid sequence 32 encoding the photosensitive transcription factor 9 is induced or stimulated (upregulated) by illuminating the cell with light (λ = 465 nm) that triggers the binding of the photosensitive transcription factor 9 to the transcription factor binding site 35 of the regulatory sequence 34.

Illumination of the cell with blue light (e.g., λ = 465 nm) triggers dimerization of the photosensitive transcription factor 9, allowing the resulting dimers 14 to bind to the transcription factor binding site 35. These structural changes (described by Motta-Mena et al., 2014) ensure that the gene expression controlling domain 10 is placed in the vicinity of the first promoter 33 which is activated by the transcriptional activation domain. Accordingly, transcription of the first nucleic acid sequence 32 encoding the photosensitive transcription factor 9 is rapidly induced and self-stimulated by the first promoter 33 upon switching on a suitable light source emitting blue light. If this light source is switched off, the structural changes described above spontaneously reverse in the dark so that binding of the dimer of the transcription factor 14 to the first transcription factor binding site 35 and expression of the first nucleic acid sequence 32 is stopped immediately. Fast and precise optogenetic control of the production of the photosensitive transcription factor 9 can thus be ensured using this advantageous optogenetic switch according to the invention, wherein the expression cassette 31 represents a self-stimulating photoswitch providing a sufficient number of photosensitive molecules within the cell only upon illumination but can be rapidly stopped by switching-off the light. Accordingly, as the expression of the photosensitive transcription factor 9 can be limited, possible cytotoxic effects can be eliminated, or at least reduced and higher dynamic ranges can be ensured though light-dependent signal amplification via self-regulation of the photosensitive transcription factor.

The expression cassette 31 can be used, for example, with the expression systems 1 and 20 according to Figures 1 and 2, respectively, replacing the first expression cassette 2. Thus, both, the photoswitch (comprising expression cassette 31) and the genes of interest (second, third, and optionally further first nucleic acid sequences), are expressed to a very limited extent without exposure to light of the respective wavelength, leading to less cytotoxicity and higher dynamic range.

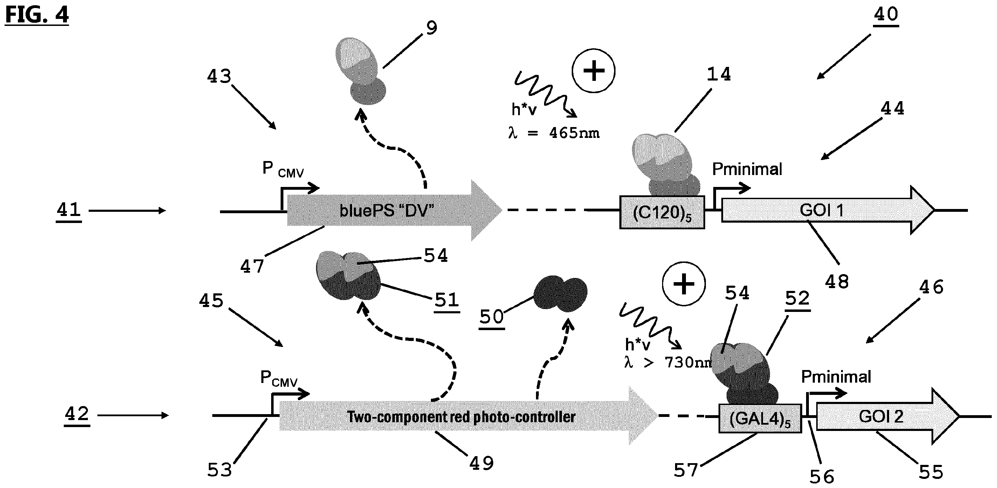

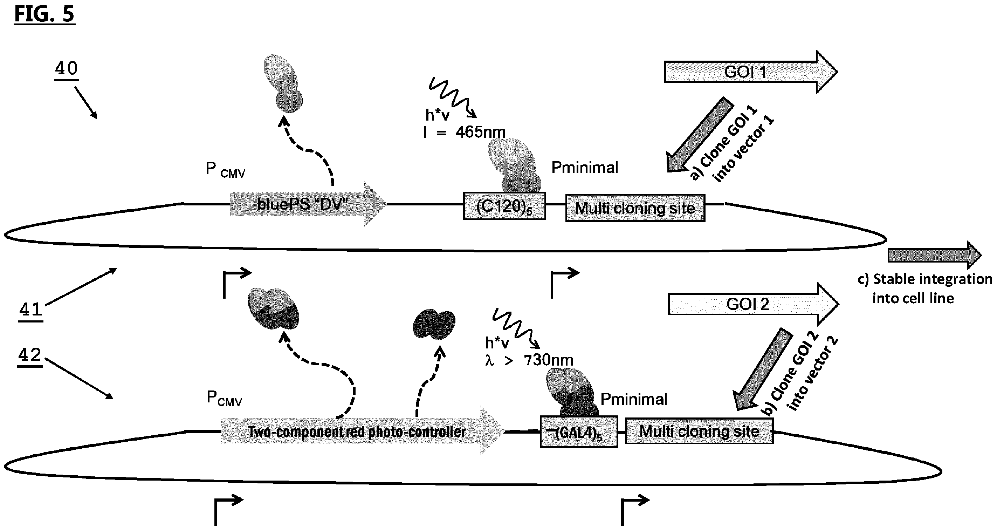

**Figures 4** and **5** show an exemplary embodiment of an expression system 40 according to the invention comprising two genetically engineered constructs 41, 42 including four expression cassettes 43, 44, 45, 46 for balancing or independently temporally controlling several components of a complex protein product (protein of interest). The genetically engineered constructs 41, 42 can be used in a method for generating a stable cell line, for example, for the production of biopharmaceuticals. The expression system 40 comprises genetically engineered constructs 41, 42 (e.g., plasmid vectors), each comprising at least one multiple cloning site, into which at least one gene of interest ("GOI 1" and "GOI 2", respectively) is cloned. Each gene of interest encodes at least one subunit of the complex protein of interest. The first construct 41 comprises two expression cassettes 43, 44 that are similar to the expression cassettes 2 and 3 according to Figures 1 and 2. Accordingly, the first expression cassette 43 comprises a first nucleic acid sequence 47 ("bluePS "DV"") encoding a photosensitive transcription factor 9 and thus also represents a constitutively expressed photoswitch providing a constantly sufficient level of photosensitive transcription factors 9 within a cell. The second expression cassette 44 comprises a second nucleic acid sequence 48 ("GOI 1") encoding at least one first subunit of the protein of interest. The first and the second expression cassette 43, 44 and their respective effects within the cell are described in detail above with reference to the expression cassettes 2 and 3 according to Figure 1, respectively.

The second construct 42 comprises a different first expression cassette 45 comprising a first nucleic acid sequence 49 ("Two-component red photo-controller") encoding two components 50, 51 of a photosensitive transcription factor 52 and thus also represents a constitutive photoswitch providing a constantly sufficient level of photosensitive transcription factors 52 within the cell. To this end, the first nucleic acid sequence 49 is operably linked to a first promoter 53 being disposed upstream of the first nucleic acid sequence 49. The first promoter 53 may be a constitutive promoter such as the cytomegalovirus promoter ("P_{CMV}"), allowing for continual transcription of the first nucleic acid sequence 49. The first nucleic acid sequence 49 encodes two components 50, 51 of the photosensitive transcription factor 52, wherein the first component 50 may comprise a phytochrome B and a DNA binding domain, and the second component 51 may comprise a phytochrome-interacting domain and a gene expression controlling domain 54 comprising a transcriptional activation domain such as VP16, VP64, p65, RTA or any combination thereof.

The second construct 42 further comprises a third expression cassette 46 comprising a third nucleic acid sequence 55 ("GOI 2") encoding at least one second subunit of the protein of interest. The third nucleic acid sequence 55 is operably linked to a third promoter 56 such as an inducible minimal promoter ("Pₘᵢₙᵢₘₐₗ") which is disposed upstream of the third nucleic acid sequence 55. The third promoter 56 is operably linked to a transcription factor binding site 57 ("(GAL4)s") arranged upstream of the third promoter 56. For example, an optogenetic switch as described by Redchuck et al. (2018) may be used in this embodiment of the invention. That is, upon far-red light (λ > 730 nm) stimulation, phytochrome B (PhyB), which remains in its inactive form in the dark, undergoes a conformational change, turning into the active form. The active PhyB heterodimerizes with the phytochrome-interacting domain and thus a complex of the two components 50, 51 is formed, said complex representing the (active) photosensitive transcription factor 52, wherein the (GAL4) DNA binding domain and the transcriptional activation domain are brought together at the transcription factor binding site 57, triggering transcription of the gene of interest ("GOI 2"). Upon red (λ = 630 nm) illumination, the complex dissociates, halting transcription immediately (Redchuck et al., 2018). Accordingly, transcription of the third nucleic acid sequence 55 encoding the at least one second subunit of the protein of interest can be rapidly induced by the third promoter 56 upon switching on a suitable light source emitting far-red light. If this light source is switched off, the structural changes described above spontaneously reverse in the dark so that expression of the third nucleic acid sequence 55 is stopped immediately. Fast and precise optogenetic control of the production of a complex protein product such as a biopharmaceutical can thus be ensured using the method and expression system according to the invention.

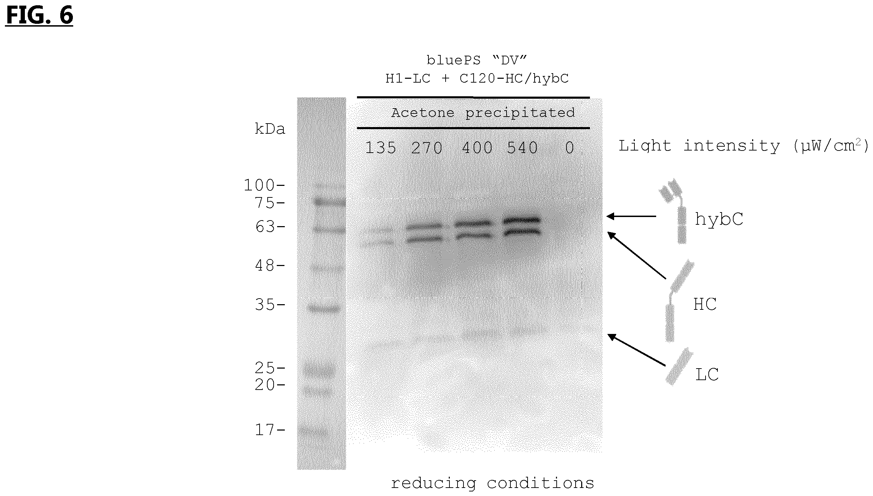

**Figure** 6 shows the control of product composition and output with light, in particular a light intensity-dependent increase in bispecific antibody (bsAb) expression/secretion upon blue light stimulation. The Western blot (WB) analysis demonstrates the expression/secretion of bispecific antibodies (bsAb) regulated by different light intensities. The heavy chain (HC, 52 kD) and the single chain variable fragment (hybC; 54 kD) were expressed in a light dependentendent-manner using the expression system 20 according ti Figure 2 including the blue photoswitch ("bluePS"). The light chain (LC; 25 kD) was expressed constitutively under the control of the H1 promoter. The WB shows the bsAb under reducing conditions which results in the disassembly of the bsAb complex into the individual light (LC), heavy (HC) and hybrid (hybC) chains. Accordingly, it can be demonstrated that bispecific antibody sidechains can be induced with light and different light intensities can be used to titrate sidechains individually.

### Material and Methods:

800,000 HEK293T cells/well were seeded in a 6-well plate in 1265 µl OptiMEM (Gibco). After 4 h cells were transfected with 2.5 µg total DNA/well using polyethyleneimine (PEI) in a ratio 1:2 (DNA:PEI). The following genes were transfected: the blue photoswitch bluePS "DV" (encoding the light-oxygen-voltage-sensing domain EL222, a transcriptional activation domain comprising VP64, p65, and RTA, and two nuclear localization signals, preferably the amino acid sequence according to SEQ ID NO: 1), the constitutively expressed light chain pH1-LC, and the light-dependent heavy (C120-HC) and hybrid (C120-hybC) chains in a ratio 3:1:1:1. Cells were excited 24 h after transfection for 24 h at indicated intensities with blue light (λ = 465 nm). Cell culture supernatant was collected, and cellular debris was removed. Proteins were precipitated by adding three times the volume of acetone overnight at -20 °C, followed by centrifugation for 10 min at 4,000 g and 4 °C. Pellets were airdried for 30 min and resuspended in Tris/1% SDS (pH 6.8). Protein concentrations were determined via BCA assay. Loading dye (200 mM Tris/HCl pH 6.8, 8% (w/v) SDS, 50% (v/v) glycerin, 4% (v/v) mercaptoethanol, 0.04% bromophenol blue) was added to the samples. Samples were denatured for 5 min at 95 °C and loaded on a 4-20% SDS-PAGE. Proteins were blotted on PVDF membranes (Merck Millipore) using a semi-dry blotter (BioRad). Membranes were blocked in 5% BSA (Sigma-Aldrich). Produced antibodies were detected with a goat-anti-human IgG antibody coupled to HRP (1 :5000) and ECL (Perkin Elmer).

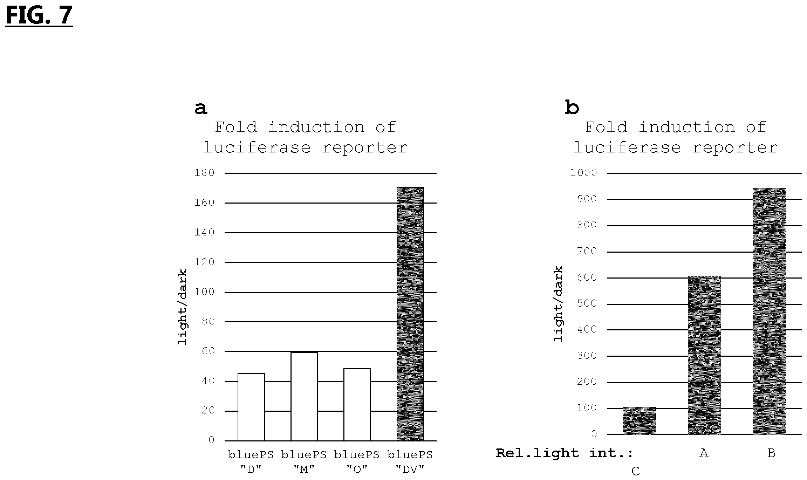

**Figure 7** shows the stimulating effect of a first expression cassette according to the invention (blue-sensitive photoswitch bluePS "DV"), resulting in up to almost 1000-fold induction. The data represent the means of three samples luminescence values light-exposed or kept in the dark samples, plotted against each other. Each value is the result of the firefly luminescence normalized by its corresponding renilla luminescence. It is demonstrated that the photoswitch bluePS "DV" according to the invention shows unprecedented dynamic range.

Left diagram (a): Comparison of the protein expression levels mediated by different versions ("D", "M", "O", and "DV") of the first expression cassette according to the invention (blue-sensitive photoswitch "bluePS "DV"") tested during the development of an improved photo-controller (bluePS "DV"). Cells were excited using constant blue light (λ = 465 nm) for 9 h at the intensity of 400 µW/cm².

Right diagram (b): Light intensity dependency of the protein expression levels mediated by the improved blue-sensitive photoswitch bluePS "DV". Cells were excited using constant blue light (λ = 465 nm) for 9 h at the intensity of 350 µW/cm² (A), 870 µW/cm² (B), and 1570 µW/cm² (C). The expression level mediated by bluePS "DV" increases with increasing light intensity, demonstrating that the induction of reporter gene expression is a light-dependent process.

### Material and Methods:

50,000 HEK293T cells/well were seeded in 50 µl DMEM+10% FCS/well in two 96-well plates, one exposed to the blue light and one kept in the dark. 1 h after seeding, cells were transfected with 0,1 µg total DNA/well using Lipofectamine 3000 in a ratio of 1:3 (DNA:Lipofectamine). The following genes were transfected in the ratio 5:1:0,1: as blue-sensitive photoswitch, the bluePS "DV"; as light-sensitive luminescent reporter, (C120)s-Firefly Luciferase; as constitutive luminescent reporters, SV40p-Renilla Luciferase for the normalization of the firefly measures, and HSV TPk/SV40p/CMVp/H1p-Firefly Luciferase as references for constitutive protein expression. Cells were excited with constant blue light (λ = 465 nm) for 9 h, starting 8 h after transfection, and the intensity of 400 µW/cm². Terminating the light excitation protocol, the dual luciferase assay was performed on the cells following the instruction of the Dual-Glo^{®} kit (Promega; Cat. Nr. E2940). As the first step, the culture medium was removed and substituted by 40 µL /well of Opti-MEM. To each plate well was then added an equal volume of Dual-Glo^{®} Reagent and let it incubate for 30 minutes, to allow the cell lysis to occur. At the end of the incubation, the firefly luminescence was measured with the FLUOstar Omega microplate reader. Afterward, the Renilla luminescence was activated and the firefly luminescence was quenched by adding a volume equal to the original culture medium volume of Dual-Glo^{®} Stop & Glo^{®} Reagent. Again, the incubation time was of 30 minutes, at the end of which the Renilla luminescence was measured. The ratio between the experimental reporter Firefly and the control reporter Renilla luminescence allowed to normalize the measured values.

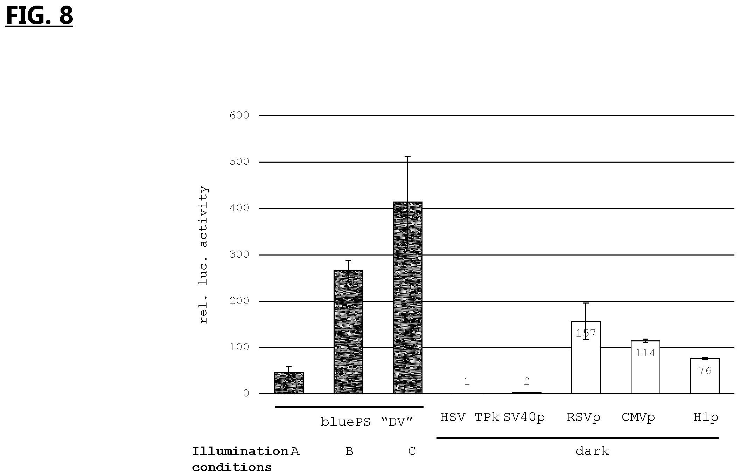

**Figure 8** shows a comparison of the protein expression levels mediated by an exemplary embodiment of a first expression cassette according to the invention (blue-sensitive photoswitch bluePS "DV") and different constitutive promoters ("HSV", "TPk", "SV40p", "RSVp", "CMVp", and "H1p"), demonstrating that this "DV" photo-controller in combination with the (C120)₅ transcription factor binding element has superior strength. Cells were excited using constant blue light (λ = 465 nm) for 9 h at the intensities of 350 µW/cm² (A), 870 µW/cm²(B) and 1570 µW/cm² (C). The data represent the means of three samples of firefly luminescence values light-exposed (for bluePS "DV" samples) or kept in the dark (for the constitutive promoters' samples), normalized by their corresponding Renilla luminescence. It is demonstrated that the photoswitch bluePS "DV" according to the invention shows a fast and tunable response to illumination, allows for dynamic gene control, and at least matches or even outperforms state-of-the-art industry expression systems.

### Material and Methods:

50,000 HEK293T cells/well were seeded in 50 µl DMEM+10% FCS/well in two 96-well plates, one exposed to the blue light and one kept in the dark. 1 h after seeding, cells were transfected with 0,1 µg total DNA/well using Lipofectamine 3000 in a ratio of 1:3 (DNA:Lipofectamine). The following genes were transfected in the ratio 5:1:0,1: as blue-sensitive photoswitch, the bluePS "DV"; as light-sensitive luminescent reporter, (C120)s-Firefly Luciferase; as constitutive luminescent reporters, SV40p-Renilla Luciferase for the normalization of the firefly measures and HSV TPk/SV40p/CMVp/H1p-Firefly Luciferase as references for constitutive protein expression. Cells were excited with constant blue light (λ = 465 nm) for 9 h, starting 8 h after transfection, and at the intensity of 350 µW/cm² (A), 870 µW/cm² (B) and 1570 µW/cm² (C).. Terminating the light excitation protocol, the dual luciferase assay was performed on the cells following the instruction of the Dual-Glo^{®} kit (Promega; Cat. Nr. E2940). As first step, the culture medium was removed and substituted by 40 µL /well of Opti-MEM. To each plate well was then added an equal volume of Dual-Glo^{®} Reagent and let it incubate for 30 minutes, to allow the cell lysis to occur. At the end of the incubation, the firefly luminescence was measured with the FLUOstar Omega microplate reader. Afterward, the Renilla luminescence was activated and the firefly luminescence was quenched by adding a volume equal to the original culture medium volume of Dual-Glo^{®} Stop & Glo^{®} Reagent. Again, the incubation time was of 30 minutes, at the end of which the Renilla luminescence was measured. The ratio between the experimental reporter firefly and the control reporter Renilla luminescence allowed to normalize the measured values.

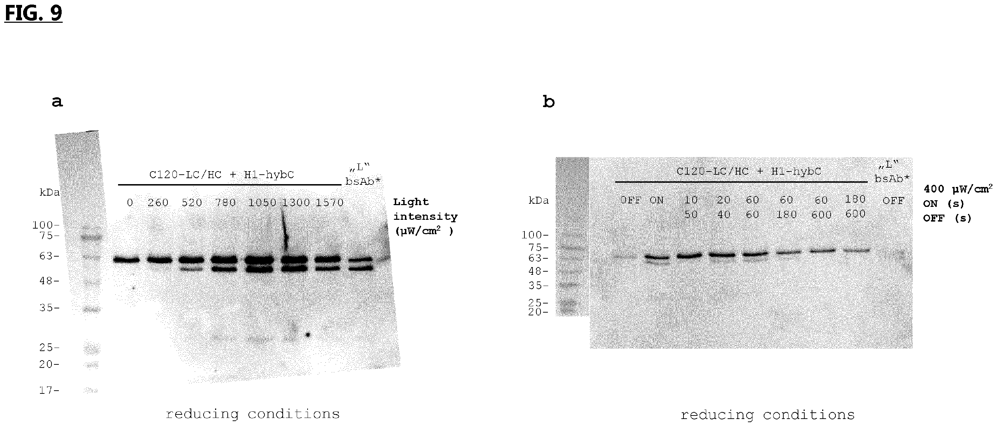

**Figure 9** shows the results of Western blot analyses demonstrating light dose-dependent expression/secretion of bispecific antibodies (bsAb), in particular titration of an antibody composition for better output. The light (LC) and the heavy (HC) chains are expressed in a light-dependent manner, the hybrid chain (hybC) is constitutively expressed (Light chain (LC): ca. 25 kDa, heavy chain (HC): ca. 52 kDa, hybrid chain (hybC): ca. 54 kDa).

Left picture (a): Western blot (WB) demonstrating a light intensity-dependent increase in LC and HC expression/secretion upon blue light stimulation, while the hybC expression is not influenced by light excitation.

Right picture (b): Western blot (WB) demonstrating a light pulse-dependent increase in LC and HC expression/secretion upon blue light stimulation, while the hybC expression is not influenced by light excitation. WB shows bsAb under reducing conditions which results in the disassembly of the bsAb complex into the individual light (LC), heavy (HC) and hybrid (hybC) chains.

### Material and Methods:

140,000 HEK293T cells/well were seeded in a 24-well plate in 300 µl OptiMEM. After 4 h cells were transfected with 0.5 µg total DNA/well using PEI in a ratio 1:2 (DNA:PEI). The following genes were transfected: the blue photoswitch bluePS "DV" (encoding the light-oxygen-voltage-sensing domain EL222, a transcriptional activation domain comprising VP64, p65, and RTA, and two nuclear localization signals, preferably the amino acid sequence according to SEQ ID NO: 1), the consitutively expressed hybrid chain pH1-hybC and the light-dependent light (C120-LC) and heavy (C120-HC) chains in a ratio 3:1:1:1. Cells were excited 24 h after transfection for 24 h continuously at indicated intensities (left) or at indicated pulses at intensity of 400 µW/cm² (right) with blue light (λ = 465 nm). Cell culture supernatant was collected, and cellular debris was removed. Loading dye (200 mM Tris/HCl pH 6.8, 8% (w/v) SDS, 50% (v/v) glycerin, 4% (v/v) mercaptoethanol, 0.04% bromophenol blue) was added to the samples. Samples were denatured for 5 min at 95 °C and loaded on a 4-20% SDS-PAGE. Proteins were blotted on PVDF membranes (Merck Millipore) using a semi-dry blotter (BioRad). Membranes were blocked in 5% BSA (Sigma-Aldrich). Produced antibodies were detected with a goat-anti-human IgG antibody coupled to HRP (1:5000) and ECL (Perkin Elmer).

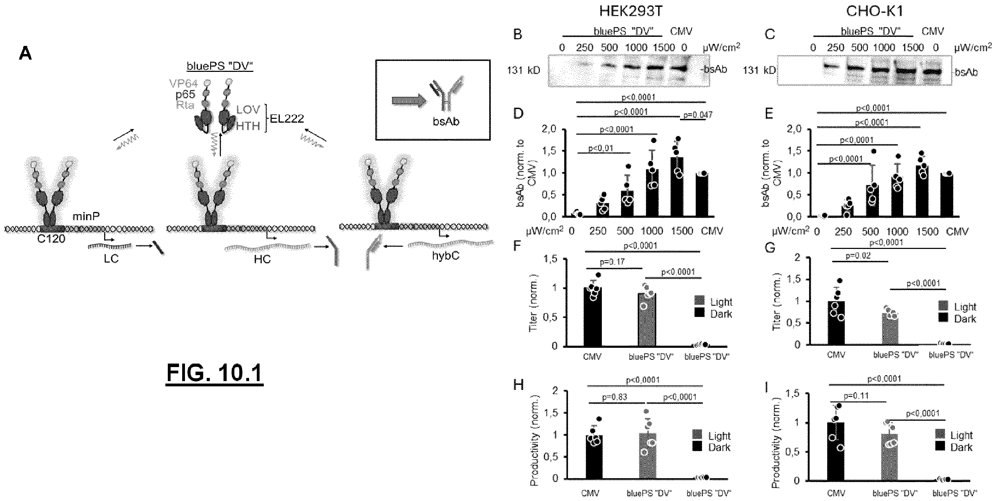

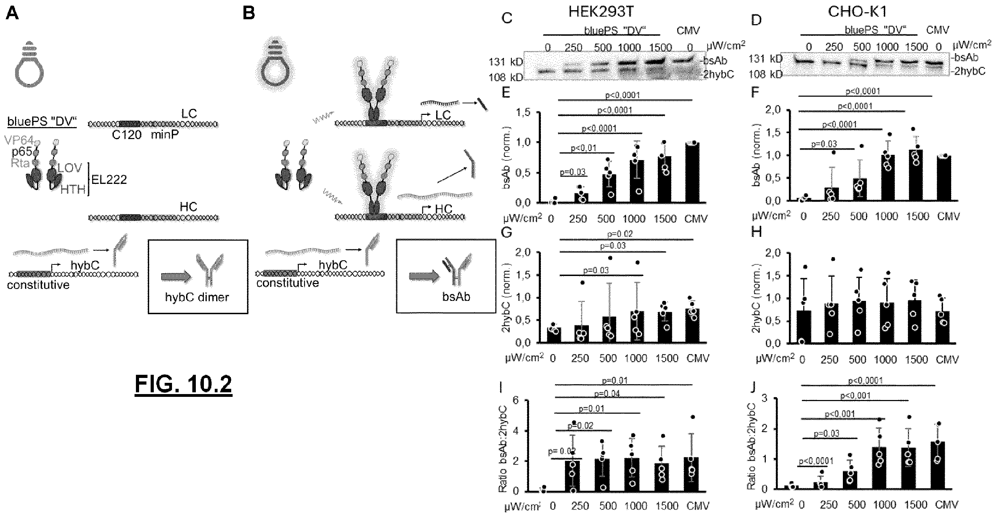

**Figure 10** shows the result of a Western blot (WB) analysis under non-reducing conditions demonstrating light intensity-dependent increase in bispecific antibody (bsAb) expression/secretion upon blue light stimulation.

**Figure 10****.****1** shows light-dependent influence of expression of all side chains on bsAb complex formation HEK293T and CHO-K1. **A)** The pDEL-VPR is activated by blue light excitation (λ = 465 nm) and in turn dimerizes and binds to the C120 sequence. Subsequently, the light (LC) heavy (HC) and single variable fragment (hybC) chains are expressed in a light dose-dependent manner, followed by their assembly into bsAb in the ER. The bsAb was either expressed in HEK293T **(B)** or CHO-K1 **(C)** either light-dependently under the control of the C120 promoter or constitutively under the control of the CMV promoter. The light-dependent conditions were excited at different intensities (µW/cm²) for 24 h (always ON). The samples were analyzed on a Western blot (WB) under non-reducing conditions using an anti-human IgG1 detection antibody coupled to HRP. The bsAb bands of the WB for the HEK293T and CHO-K1 were quantified in **D)** and **E),** respectively. **F)** and **G)** show the fold change of bsAb titer of the light-induced (C120 light; 1500 µW/cm²), non-induced (C120 dark; 0 µW/cm²) or the constitutive (CMV) conditions after 24 h expression in HEK293T or CHO-K1, respectively, quantified by ELISA. The values were normalized to the CMV samples. **H)** and **I)** show the fold change of bsAb productivity of the experiment presented in **F-G)** in HEK293T and CHO-K1, respectively. The values were normalized to the CMV samples. **D-I)** Means of biological independent samples ± STD are presented as bars, dots indicate individual values of each sample. Statistical analysis was performed using one-way ANOVA with Holm-Sidak post-hoc test. Plots contain five (n=5) in **C-E)** and six (n=6) independent samples in **F-I).**

**Figure 10****.****2** shows light-dependent influence of expression of the light (LC) and heavy (HC) chains on bsAb complex formation HEK293T and CHO-K1. **A)** In the dark, the pDEL-VPR stays inactive, therefore, the light-dependent expression of the light (LC) and heavy (HC) chain is not induced, while the single chain variable fragment (hybC) in constitutively expressed. Consequently, in the absence of the LC and HC, the hybC is assembled in dimers. **B)** The pDEL-VPR is activated by blue light excitation (λ = 465 nm) and in turn dimerizes and binds to the C120 sequence. Subsequently, the LC and HC are expressed in light intensity-dependent manner. Additionally, the hybC is expressed constitutively. Consequently, in the light the trimeric bsAb is assembled. The bsAb was expressed in HEK293T (C) or CHO-K1 **(D)** either partially light-dependently (light-dependent LC/HC + constitutive hybC) under the control of the C120 promoter or all chains constitutively under the control of the CMV promoter. The light-dependent conditions were excited at different intensities (µW/cm²) for 24 h (always ON). The different bsAb complexes were analyzed on a Western blot (WB) under non-reducing conditions using an anti-human IgG1 detection antibody coupled to HRP. The bsAb bands of the WB for the HEK293T and CHO-K1 were quantified in **E)** and **F),** respectively. The bands of hybC dimers for the HEK293T and CHO-K1 were quantified in **G)** and **H),** respectively. The ratio of bsAb:2hybC dimers for the HEK293T and CHO-K1 were quantified in **I)** and **J),** respectively. **E-J)** Means of biological independent samples ± STD are presented as bars, dots indicate individual values of each sample. Statistical analysis was performed using ANOVA with Holm-Sidak post-hoc test. Plots contain five (n=5) independent samples.

### Material and Methods:

800,000 HEK293T cells/well were seeded in a 6-well plate in 1265 µl OptiMEM. After 4 h cells were transfected with 2.5 µg total DNA/well using PEI in a ratio 1:2 (DNA:PEI). Following genes were transfected: pDEL-VPR, pH1-LC, C120-HC and C120-hybC in a ratio 3:1:1:1. Cells were excited 24 h after transfection for 24h at indicated intensities with blue light (λ = 465 nm). Cell culture supernatant was collected, and cellular debris was removed. Proteins were precipitated by adding three times the volume of acetone overnight at -20 °C, followed by centrifugation for 10 min at 4,000 g and 4 °C. Pellets were airdried for 30 min and resuspenden in Tris/1% SDS (pH 6.8). Protein concentrations were determined via BCA assay. Loading dye (200 mM Tris/HCl pH 6.8, 8% (w/v) SDS, 50% (v/v) glycerin, 4% (v/v) mercaptoethanol, 0.04% bromophenol blue) was added to the samples. Samples were denatured for 5 min at 95 °C and loaded on a 4-20% SDS-PAGE. Proteins were blotted on PVDF membranes (Merck Millipore) using a semi-dry blotter (BioRad). Membranes were blocked in 5% BSA (Sigma-Aldrich). Produced antibodies were detected with a goat-anti-human IgG antibody coupled to HRP (1:5000) and ECL (Perkin Elmer).

Light chain (LC) ca. 25 kD, heavy chain (HC) ca. 52 kD, single chain fragment (hybC) ca. 54 kD

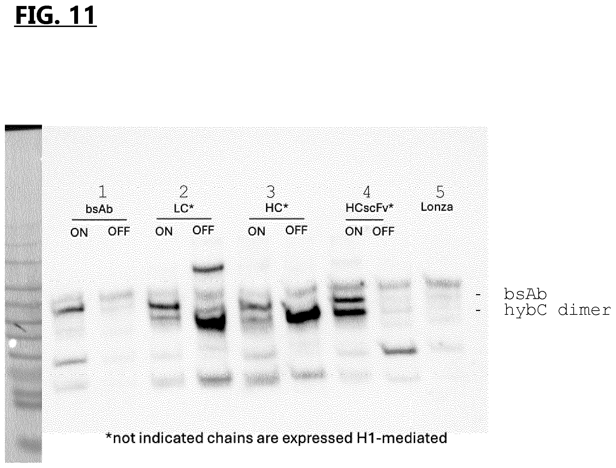

**Figure 11** shows the result of a western blot (WB) under non-reducing conditions demonstrating that expression/secretion of bispecific antibodies (bsAb) complexes is regulated by the presence/relative concentrations and/or temporally controlled expression of different amounts of light-dependent chains.

Figure 11 demonstrates that expression/secretion of bispecific antibodies (bsAb) complexes is regulated by different combination of light-dependent chains and the expression of the main product bsAB and by-products are influenced by the relative amounts and/or timing of sidechains expressed. bsAb complexes resulted from light-dependent expression of 1) all three bsAb chains, 2) the LC, 3) the HC and 4) the hybC in the light (24 h always ON, 1500 µW/cm²) or 5) constitutive expression.

With light-dependent expression of the light chain (LC, lanes 2) the formation of by-products, in particular the HCcsFv dimer is largely favored, while with expression of the light chain (LC) initiated 24 h after transfection with the constitutively expressed heavy chain (HC) and the hybC chain, the products are shifted in favor of the intended product bsAb. Similar effects can be seen in case of the light-induced heavy chain (lanes 3), while with full light-induction of the hybC chain still a significant amount of hybC dimer by-product can be detected (lanes 4). The latter might be due to overexpression of hybCrelative to the LC and HC chains. Thus, exact balancing and/or timing of expression the three bsAb components has a striking effect on the formation of intended product vs by-products, their relative concentrations, and thus on unpurified product quality, which as an impact on necessary purification efforts, yields of purified product and thus in sum production costs per unit of bsAb product.

### Material and Methods:

140,000 HEK293T cells/well were seeded in a 24-well plate in 400 µl OptiMEM. After 4 h cells were transfected with 0.5 µg total DNA/well using PEI in a ratio 1:2 (DNA:PEI). Following genes were transfected: 1) pDEL-VPR + C120-LC + C120-HC + C120-hybC, 2) pDEL-VPR + C120-LC + H1-HC + H1-hybC, 3) pDEL-VPR + H1-LC + C120-HC + H1-hybC, 4) pDEL-VPR + H1-LC + H1-HC + C120-hybC in a ratio 3:1:1:1 or 5) CMV-LC + CMV-HC + CMV- hybC. Cells were excited 24 h after transfection for 24 h at indicated intensities with blue light (λ = 465 nm). Cell culture supernatant was collected, and cellular debris was removed. Non-reducing loading dye (200 mM Tris/HCl pH 6.8, 8% (w/v) SDS, 50% (v/v) glycerin, 0.04% bromophenol blue) was added to the samples. Samples were denatured for 5 min at 95 °C and loaded on a 4-20% SDS-PAGE. Proteins were blotted on PVDF membranes (Merck Millipore) using a semi-dry blotter (BioRad). Membranes were blocked in 5% BSA (Sigma-Aldrich). Produced antibodies were detected with a goat-anti-human IgG antibody coupled to HRP (1:5000) and ECL (Perkin Elmer).

Molecular weights: Light chain (LC) ca. 25 kD, heavy chain (HC) ca. 52 kD, single chain fragment (hybC) ca. 54 kD

### Non-patent literature:

Carver J, Ng D, Zhou M, et al. Maximizing antibody production in a targeted integration host by optimization of subunit gene dosage and position. Biotechnol Progress. 2020; 36:e2967. https://doi.org/10.1002/btpr.2967
Jan N Hansen, Fabian Kaiser, Christina Klausen, Birthe Stüven, Raymond Chong, Wolfgang Bönigk, David U Mick, Andreas Möglich, Nathalie Jurisch-Yaksi, Florian I Schmidt, Dagmar Wachten (2020) Nanobody-directed targeting of optogenetic tools to study signaling in the primary cilium eLife 9:e57907; https://doi.org/10.7554/eLife.57907
Motta-Mena L.B., Reade A., Mallory M.J., Glantz S., Weiner O.D, Lynch K.W., Gardner K.H. An optogenetic gene expression system with rapid activation and deactivation kinetics. Nat Chem Biol. 10(3):196-202 (2014). https://doi: 10.1038/nchembio. 1430
Pastrana, E. Optogenetics: controlling cell function with light. Nat Methods 8, 24-25 (2011). https://doi.org/10.1038/nmeth.f.323
Redchuk, T., Kaberniuk, A. & Verkhusha, V. Near-infrared light-controlled systems for gene transcription regulation, protein targeting and spectral multiplexing. Nat Protoc 13, 1121-1136 (2018). https://doi.org/10.1038/nprot.2018.022
Rost B.R., Schneider-Warme F., Schmitz D., Hegemann, P. Optogenetic Tools for Subcellular Applications in Neuroscience. Neuron 96(3), 572-603 (2017). https://doi.org/10.1016/j.neuron.2017.09.047
Shimizu-Sato, S., Huq, E., Tepperman, J. et al. A light-switchable gene promoter system. Nat Biotechnol 20, 1041-1044 (2002). https://doi.org/10.1038/nbt734

## Claims

1. Method for producing at least one biologically active molecule, in particular a biotherapeutic, by optogenetic control of gene expression in at least one eukaryotic or prokaryotic cell, wherein the biologically active molecule comprises at least one protein of interest comprising at least two different subunits (5, 6, 23, 24, 25), said method comprising:
- introducing into the cell at least one first expression cassette (2, 31, 43, 45) comprising at least one first nucleic acid sequence (7, 32, 47, 49) linked to a first promoter (8, 33, 53), wherein the first nucleic acid sequence (7, 32, 47, 49) encodes at least one photosensitive transcription factor (9, 52),
- introducing into the cell a second expression cassette (3, 44) comprising at least one second nucleic acid sequence (11, 48) encoding a first (5, 23) of the at least two subunits (5, 6, 23, 24, 25) of the protein of interest and being operably linked to a second promoter (12), the second promoter (12) being operably linked to at least one transcription factor binding site (13), and
- introducing into the cell a third expression cassette (4, 21, 46) comprising at least one third nucleic acid sequence (15, 26, 55) encoding at least a second (6, 24) of the at least two subunits (5, 6, 23, 24, 25) of the protein of interest and being operably linked to a third promoter (16, 27, 56),
wherein expression of the second nucleic acid sequence (11, 48) is controlled through activation or repression of the second promoter (12) by illuminating the cell with light comprising at least one wavelength that triggers at least one structural change of the photosensitive transcription factor (9).

2. Method according to claim 1, the structural change causes binding or release of the photosensitive transcription factor (9) to/from the transcription factor binding site (13), or wherein the structural change causes nuclear trafficking of the photosensitive transcription factor (9).

3. Method according to claim 1 or 2, wherein the photosensitive transcription factor (9, 52) comprises at least one gene expression controlling domain (10, 54) and/or forms a complex with at least one gene expression controlling domain (10, 54), wherein the gene expression controlling domain (10, 54) comprises a transcriptional activation domain or a transcriptional repression domain.

4. Method according to any one of claims 1 to 3, wherein the first promoter (8, 53) is a constitutive promoter or wherein the first promoter (33) is operably linked to at least one regulatory sequence (34), wherein the regulatory sequence (34) comprises at least one transcription factor binding site (35), wherein the first promoter (33) and thus expression of the first nucleic acid sequence (32) encoding the photosensitive transcription factor (9) is upregulated by illuminating the cell with light comprising at least one wavelength that triggers binding of the photosensitive transcription factor (9) to a transcription factor binding site (35) of the regulatory sequence (34).

5. Method according to any one of claims 1 to 4, wherein the third promoter (16) is a constitutive promoter or wherein the third promoter (27, 56) is operably linked to at least one transcription factor binding site (28, 57), wherein expression of the third nucleic acid sequence (26, 55) is controlled through activation or repression of the third promoter (27, 56) by illuminating the cell with light comprising at least one wavelength that triggers at least one structural change of said photosensitive transcription factor (9, 52) or another photosensitive transcription factor.

6. Method according to claim 5, wherein the structural change causes binding or release of the photosensitive transcription factor (9, 52) to/from the transcription factor binding site (28, 57), binding or release between at least one DNA binding subunit and at least one transcription activating subunit of the transcription factor, or wherein the structural change causes nuclear trafficking of the photosensitive transcription factor (9, 52).

7. Method according to any one of claims 1 to 6, wherein at least one additional expression cassette (22) is introduced into the cell, the additional expression cassette (22) comprising at least one further nucleic acid sequence (29) encoding at least one further subunit (25) of the at least two subunits (5, 6, 23, 24, 25) of the protein of interest and being operably linked to a further promoter sequence (30).

8. Method according to claim 7, wherein the further promoter sequence (30) comprises a constitutive promoter or wherein the further promoter sequence is operably linked to at least one transcription factor binding site, wherein expression of the further nucleic acid sequence is controlled through activation or repression of the further promoter sequence by illuminating the cell with light comprising at least one wavelength that triggers at least one structural change of said photosensitive transcription factor (9, 52) or another photosensitive transcription factor.

9. Method according to claim 8, wherein the structural change causes binding or release of the photosensitive transcription factor to/from the transcription factor binding site, binding or release between at least one DNA binding subunit and at least one transcription activating subunit of the transcription factor, or wherein the structural change causes nuclear trafficking of the photosensitive transcription factor.

10. Expression system (1, 20, 40) for producing at least one biologically active molecule, in particular a biotherapeutic, by optogenetic control of gene expression in at least one eukaryotic or prokaryotic cell, wherein the biologically active molecule comprises at least one protein of interest comprising at least two different subunits (5, 6, 23, 24, 25), said expression system comprising:
- At least one first expression cassette (2, 31, 43, 45) comprising at least one first nucleic acid sequence (7, 32, 47, 49) linked to a first promoter (8, 33, 53), wherein the first nucleic acid sequence (7, 32, 47, 49) encodes at least one photosensitive transcription factor (9, 52) comprising a gene expression controlling domain (10, 54) and/or having a structure that enables forming a complex with at least one gene expression controlling domain (10, 54),
- a second expression cassette (3, 44) comprising at least one second nucleic acid sequence (11, 48) encoding a first (5, 23) of the at least two subunits (5, 6, 23, 24, 25) of the protein of interest and being operably linked to a second promoter (12), wherein the second promoter (12) is operably linked to at least one transcription factor binding site (13) and activatable or repressible by illumination with light comprising at least one wavelength that triggers at least one structural change of the photosensitive transcription factor (9), and
- a third expression cassette (4, 21, 46) comprising at least one third nucleic acid sequence (15, 26, 55) encoding at least a second (6, 24) of the at least two subunits (5, 6, 23, 24, 25) of the protein of interest and being operably linked to a third promoter (16, 27, 56).

11. Expression system according to claim 10, further comprising additional expression cassettes (22), each additional expression cassette (22) comprising at least one further nucleic acid sequence (29) encoding at least one further subunit (25) of the at least two subunits (5, 6, 23, 24, 25) of the protein of interest and being operably linked to a further promoter sequence (30).

12. Expression system according to claim 10 or 11, wherein the photosensitive transcription factor (9, 52) comprises at least one light-oxygen-voltage-sensing domain (LOV domain), preferably EL222, or at least one cryptochrome (CRY), or at least one phytochrome, preferably phytochrome B, and/or wherein the transcription factor binding site (13, 57) comprises at least one binding site selected from the group consisting of at least one (C120), GAL4, TetR, lexA, and lacR binding site.

13. Expression system according to any one of claims 10 to 12, wherein the gene expression controlling domain (10, 54) of the photosensitive transcription factor (9, 52) comprises at least one transcriptional activation domain, preferably selected from the group consisting of VP16, VP64, p65, RTA, and any combination thereof, or a transcriptional repression domain, preferably KRAB.

14. A kit or composition for producing at least one biologically active molecule, in particular a biotherapeutic, wherein the biologically active molecule comprises at least one protein of interest comprising at least two subunits, by optogenetic control of gene expression of at least one of the at least two subunits in at least one eukaryotic or prokaryotic cell, comprising the expression system according to any one of claims 10 to 13.

15. A photosensitive transcription factor comprising the light-oxygen-voltage-sensing domain EL222, a transcriptional activation domain comprising VP64, p65, and RTA, and two nuclear localization signals, preferably comprising the amino acid sequence according to SEQ ID NO: 1.

16. A nucleic acid molecule comprising a nucleotide sequence encoding the photosensitive transcription factor according to claims 15.
